# EUROPEAN PATENT SPECIFICATION

(11) **EP 0 582 143 B1**
(45) Date of publication and mention of the grant of the patent: **04.11.1998**
(21) Application number: 93111756.8
(22) Date of filing: 22.07.1993
(51) Int. Cl.: G01N 33/573, G01N 33/68

(54) **Methods for detecting Alzheimer's disease**
Verfahren zum Nachweis von Alzheimer-Krankheit
Méthodes pour la détection de la maladie d'alzheimer

(30) Priority: 22.07.1992 US 925594
(43) Date of publication of application: 09.02.1994
(73) Proprietor: THE McLEAN HOSPITAL CORPORATION, Belmont, MA 02178 (US)
(72) Inventor: Nixon, Ralph A., Arlington, Mass. 02174 (US); Saito, Ken-Ichi, Midori-ku, Yokohama 227 (JP)
(74) Representative: Hansen, Bernd, Dr. Dipl.-Chem.

(56) References cited:
- WO-A-93/02106
- BIOLOGICAL ABSTRACTS vol. 93, no. 1 1992, Philadelphia, PA, US; abstract no. 7719, N. IWAMOTO ET AL. 'Localization of calpain immunoreactivity in senile plaques and in neurones undergoing neurofibrillary degeneration in Alzheimer's disease.' page AB-826 ; & BRAIN RES. vol. 561, no. 1 , 1991 pages 177 - 180
- BIOLOGICAL ABSTRACTS vol. 90, no. 4 1990, Philadelphia, PA, US; abstract no. 42455, L. S. PERLMUTTER ET AL. 'Distribution of calcium-activated protease calpain in the rat brain.' page AB-807 ; & J. COMP. NEUROL. vol. 296, no. 2 , 1990 pages 269 - 276
- BIOLOGICAL ABSTRACTS vol. 90, no. 7 1990, Philadelphia, PA, US; abstract no. 78115, E. I. NILSSON ET AL. 'Calpain and calpastatin in normal and Alzheimer-degenerated human brain tissue.' page AB-735 ; & NEUROBIOL. AGING vol. 11, no. 4 , 1990 pages 425 - 432
- CHEMICAL ABSTRACTS, vol. 110, no. 17, 24 April 1989, Columbus, Ohio, US; abstract no. 152196, S. KAWASHIMA ET AL. 'Transglutaminase and calcium-protease activities in Alzheimer's disease brain.' page 531 ;column 2 ; & BIOMED. RES. vol. 10, no. 1 , 1989 pages 17 - 23
- PROCEEDINGS OF THE NATIONAL ACADEMY OF SCIENCES OF USA. vol. 90, no. 7 , 1 April 1993 , WASHINGTON US pages 2628 - 2632 K.-I. SAITO ET AL. 'Widespread activation of calcium-activated neutral proteinase (calpain) in the brain in Alzheimer disease: A potential molecular basis for neuronal degeneration.'

## Description

### Field of the Invention

The present invention relates to the field of medical diagnostics employing antibodies to screen patients for Alzheimer's disease.

### Background of the Invention

Synaptic loss and neuronal cell death correlate strongly with the degree of cognitive impairment in Alzheimer disease (Terry *et al., Ann. Neurol. 30:572-580* (1991); Hamos *et al., Ann. Neurol. 39:* 355 (1991); Dekosky & Scheff, *Ann. Neurol.* 27:457-464 (1990)). These pathologic events are not yet defined at the biochemical level although many putative etiologic factors in Alzheimer disease share in common the potential for disrupting cellular calcium homeostasis (Khachaturian, Z.S., *Aging 1(1)*:17-34 (1989)), and some evidence exists for such disruption in Alzheimer tissues (Colvin, R.A., *et al., Brain Res. 543:139-147* (1991); Peterson, C., *et al., N. Engl. J. Med.* 312(16):1063-1064 (1985); Peterson & Goldman, *Proc. Natl. Acad. Sci. USA* 83:2758-2762 (1986); Rizopoulos, E., *et al., Brain Res. Bull.* 21:825-828 (1988); Peterson, C., *et al., Neurosci. Lett.* 121:239-243 (1991); Iacopino & Christakos, *Proc. Natl. Acad. Sci. USA 87:4078-4082* (1990); Gibson, G.E., *et al., Biol. Psychiatry 22*:1079-1086 (1987)).

Calcium-activated neutral proteinases (CAN P) are a family of proteases implicated in regulating aspects of signal transduction (Pontremoli, S., *et al., Proc. Natl. Acad. Sci. USA* 87:3705-3707 (1990); Piggott, M.A., *et al., Brain* *Res*. *565*:42-47 (1991); Adunsky, A., *et al*., *J*. *Neuroimmunology 33*:167-172 (1991); Suzuki & Ohno, *Cell Struct*. *Funct*. *15*:1-6 (1990); Murachi, T., *Biochem*. *Internatl*. *18(2)*:263-294 (1989)). Limited proteolysis by CANPs is involved in regulating important enzymes, including calcium-dependent protein kinases and protein phosphatases and neurotransmitter enzymes, and in modifying the function of structural proteins of the membrane and membrane skeleton (Togari, A., *et al*., *Biochem*. *Biophys. Res*. *Commun*. *134(2)*:749-754 (1968); Kishimoto, A., *et al*., *J*. *Biol*. *Chem*. *258*:1156-1164 (1983); Tallant, E.A., *et al*., *Biochem*. *27*:2205-2211 (1988); Nixon, R.A., *Ann*. *N*.*Y*. *Acad*. *Sci*. *568*:198-208 (1989)). Massive activation of CANPs, as occurs in excitatoxicity and ischemia, induces rapid irreversible neuronal injury (Siman, R., *et al*., *J*. *Neurosci*. *9*:1579-1590 (1989); Siman, R., in *Neurotoxicity of Excitatory Amino Acids*, A. Guidotti, ed., Raven Press, New York, pp. 145-161 (1990); Lee, K.S., *et al*., *Proc*. *Natl. Acad*. *Sci*. *USA 88*:7233-7347 (1991)). However, previous reports indicate that total CANP activity is not significantly altered in Alzheimer brain (Kawashima, S., *et al*., *Biomed. Res*. *10(1)*:17-23 (1989); Mantle & Perry, *J*. *Neurol*. *Sci*. *102*:220-224 (1991); Nilsson, E., *et al*., *Neurobiol*. *Aging 11*:425-431(1990)).

CANP exists in cells principally as an inactive precursor isoform (Suzuki & Ohno, *Cell Struct*. *Funct*. *15*:1-6 (1990); Suzuki, K., *et al*., *FEBS Lett*. *220*:271 (1987)), which is activated by autoproteolytic cleavage of an amino-terminal sequence in the presence of calcium (Suzuki, K., *et al*., *J*. *Biochem*. *90*:1787-1793 (1981); Inomata, M., *et al*., *J*. *Biochem*. *98*:407-416 (1985); Zimmerman & Schlaepfer, *Biochim*. *Biophys*. *Acta 1078*:192-198 (1991); Suzuki, K., *et al*., *FEBS Lett*. *220*:271 (1987)). The action of CANP *in vivo* has been difficult to monitor by *in vitro* enzyme assay because the precursor form is also activated by the assay procedure, and activities are, in turn, influenced by instability of the enzyme as well as by various cytosolic inhibitory and activating factors (Mellgren & Murachi, eds., *Intracellular Calcium*-*dependent Proteolysis*, CRC Press, Boston, MA, pp. 1-276 (1990)).

### Summary of the Invention

To circumvent some of these problems, we identified distinct isoforms of µCANP in postmortem human brain that correspond to the autolytically activated isoform and the uncleaved precursor previously demonstrated in erythrocytes. The invention relates to the discovery that the ratio of precursor to activated isoforms of µCANP in tissues provides an index of CANP function *in vivo*.

In particular, the invention relates to a method for the detection of Alzheimer's disease in an individual, comprising
(a) obtaining a tissue sample from an individual suspected of having Alzheimer's disease; and
(b) determining the relative ratio of the active 76-kDa isoform of µCANP to the 80-kDa isoform in said tissue sample;
wherein an increased ratio of 76-kDa/80-kDa compared to a control group of individuals not having Alzheimer's disease confirms that the individual suspected of having Alzheimer's disease does have Alzheimer's disease.

The invention also relates to a method for the detection of Alzheimer's disease in an individual, comprising
(a) obtaining a tissue sample from an individual suspected of having Alzheimer's disease; and
(b) determining the relative ratio of the active 76-kDa isoform of µCANP to the total quantities of the 76-kDa, 78-kDa and 80-kDa isoforms;
wherein an increased ratio of 76-kDa/76-kDa+78-KDa+80-kDa compared to a control group of individuals not having Alzheimer's disease confirms that the individual suspected of having Alzheimer's disease has Alzheimer's disease.

The invention also relates to a kit which is useful for detecting Alzheimer's disease in a individual suspected of having the disease, comprising a carrier means having in close confinement therein one or more container means, wherein a first container means contains a detectable antibody that is specific for the active 76-kDa isoform of µCANP and the 80-kDa isoform of µCANP. Further container means may contain a detectably labelled antibody that is immunoreactive with said detectable antibody that is specific for the active 76-kDa isoform of µCANP and the 80-kDa isoform of µCANP. In addition, further container means may contain a means for separating the 76-kDa isoform of µCANP and the 80-kDa isoform of µCANP.

The invention also relates to a kit which is useful for detecting Alzheimer's disease in a individual suspected of having the disease, comprising a carrier means having in close confinement therein two or more container means, wherein a first container means contains a first detectable antibody that is immunoreactive with the 80-kDa isoform of µCANP and is not immunoreactive with the 76-kDa and 78-kDa isoforms of µCANP and a second container means contains a second detectable antibody that is immunoreactive with the 76-kDa, 78-kDa and 80-kDa isoforms of µCANP. Preferably, the first and second detectable antibodies are detectably labelled with different labels that can be discriminated on detection.

The invention also relates to a method for screening a drug for use in the treatment or prevention of Alzheimer's disease, comprising
(a) obtaining a first animal brain sample;
(b) determining the relative ratio of the active 76-kDa isoform of µCANP to the 80 kDa isoform in the first animal brain sample;
(c) incubating a second animal brain sample with an aqueous solution of Ca⁺⁺ and a drug suspected of being useful in treating or preventing Alzheimer's disease;
(d) determining the relative ratio of the active 76-kDa isoform of µCANP to the 80 kDa isoform in the tissue sample obtained in step (c);
wherein when the 76-kDa/80-kDa ratio of the animal brain sample contacted with said drug as determined in step (d) is reduced compared to the 76-kDa/80-kDa ratio obtained in step (b) confirms that the drug is useful for treating or preventing Alzheimer's disease.

The invention also relates to a method for screening a drug for use in the treatment or prevention of Alzheimer's disease, comprising
(a) obtaining a first fibroblast sample from an animal;
(b) determining the relative ratio of the active 76-kDa isoform of µCANP to the 80 kDa isoform in the first fibroblast sample;
(c) incubating a second fibroblast sample with an aqueous solution of Ca⁺⁺, a calcium ionophore, and a drug suspected of being useful in treating or preventing Alzheimer's disease;
(d) determining the relative ratio of the active 76-kDa isoform of µCANP to the 80 kDa isoform in the fibroblast sample obtained in step (c);
wherein when the 76-kDa/80-kDa ratio of the fibroblast sample contacted with said drug as determined in step (d) is reduced compared to the 76-kDa/80-kDa ratio obtained in step (b) confirms that the drug is useful for treating or preventing Alzheimer's disease.

### Description of the Figures

Fig. 1(A) depicts an immunoblot analysis of µCANP in prefrontal cortex from two control (CONT) and two Alzheimer (AD) patients.

Fig. 1(B) depicts a gel electrophoresis showing the autolysis of µCANP from human erythrocytes *in vitro*.

Fig. 2 depicts a graph showing the relationship between enzyme activity and µCANP content.

Fig. 3 depicts an immunoblot analysis of µCANP autolysis in human brain slices (prefrontal cortex).

Fig. 4 depicts a graph showing the ratio of 76 kDa/80 kDa isoforms of µCANP in prefrontal cortex from control individuals (O) and AD patients (●), plotted against postmortem interval (PMI).

Fig. 5 (Panels A, B and C) depicts bar graphs showing the µCANP isoform ratios (76 kDa/80 kDa) in brain regions from control, Alzheimer and Huntington disease (HD) brains. (A) Prefrontal cortex. (B) Putamen. (C) Cerebellum.

Fig. 6 (Panels A and B) depicts immunoblot analysis of fibroblasts from control and affected individuals from the Canadian FAD cohort. The figure shows that the ratio of activated calpain isoform (76 kDa) to latent isoform (84 kDa) is elevated in fibroblasts from phenotypically-affected individuals of the FAD Cohort (C = control).

### Description of the Preferred Embodiments

Calcium-activated neutral proteinases (CANP) are key enzymes in intra-cellular signaling cascades and are potential mediators of calcium-induced cell injury. The enzyme form requiring micromolar calcium levels (µCANP), which is enriched in neurons, has been most frequently implicated. As an index of changes in the *in vivo* activity of µCANP, the ratios of the activated isoform of µCANP to the precursor isoforms were measured immunochemically in regions of postmortem human brain. This ratio was elevated 3-fold in the prefrontal cortex from patients with Alzheimer disease but not from those with Huntington disease. Other regions of Alzheimer brain (putamen and cerebellum) where neuronal degeneration is considered minimal in Alzheimer disease also displayed significantly increased µCANP activation.

Thus, the invention relates to the discovery that elevated levels of the active isoform of µCANP is associated with Alzheimer's disease. In a first aspect, the invention relates to a method for the detection of Alzheimer's disease in an individual, comprising
(a) obtaining a tissue sample from an individual suspected of having Alzheimer's disease; and
(b) determining the relative ratio of the active 76-kDa isoform of µCANP to the 80-kDa isoform in said tissue sample;
wherein an increased ratio of 76-kDa/80-kDa compared to a control group of individuals not having Alzheimer's disease confirms that the individual suspected of having Alzheimer's disease does have Alzheimer's disease.

The tissue sample may be a brain biopsy or, more preferably, a fibroblast sample. Where a brain biopsy sample is obtained, it preferably obtained from the frontal cortex, putamen or cerebellum. When obtained from the frontal cortex, it is possible to discriminate between Alzheimer's disease and Huntington's disease. When nervous system tissue is analyzed according to the present invention, about 0.1 to about 0.5 g of tissue is needed to carry out the analysis.

Most preferably, a sample of fibroblasts is obtained from an individual suspected of having Alzheimer's disease. Fibroblasts may be obtained from individuals according to the procedure described by Willers, I. *et al., Pathobiology* 59:357-360 (1991). *See also* Ham and McKeehan, *In Vitro 14*:11-22 (1978), who teach methods for growing fibroblasts in cell culture. About 0.05 to about 0.1 g of fibroblasts is needed to carry out the analysis.

After obtaining the tissue sample, it is homogenized in a physiological buffer, centrifuged and the supernatant tested for the 76-kDa and 80-kDa isoforms of µCANP. Preferably, the supernatant is first subjected to fractionation to remove potential interfering substances. This may be carried out by applying the supernatant to a DEAE-cellulose column, and eluting the column with a buffer containing increase amounts of a salt such as potassium chloride.

The fractions eluting off the column may be subject to electrophoresis to separate the 76-kDa, 78-kDa and 80-kDa isoforms, followed by electrotransfer to a membrane (e.g., nitrocellulose) and immunoblot analysis with detectable antibodies that are immunoreactive with one or more of the 76-kDa, 78-kDa and 80-kDa isoforms. The detectable antibody may be detectably labelled as described herein or may be detected by contacting with a second antibody that is immunoreactive therewith, e.g. where the first antibody is from a first animal species and the second antibody is from a second animal species and is immunoreactive with the antibodies from the first animal species. Antibodies which are immunoreactive with the 76-kDa, 78-kDa and 80-kDa isoforms, are taught, for example, by Samis, J.A., *et al., Biochem. J. 246*:481-488 (1987); and Elce, J.S., *et al., Biochem. J. 261*:1039-1042 (1989). It is also possible to employ antibodies that are selective for only the 76-kDa isoform. *See* Saido, T.C. *et al., J. Biochem*. *111*:81-86 (1992).

More preferably, the ratio of 76-kDa and 80-kDa isoforms is determined with detectably labelled antibodies that discriminate between the 80-kDa isoform and the 76-kDa and 78-kDa isoforms. When the 80-kDa isoform is converted to the 76-kDa and 78-kDa isoforms by proteolysis, the N-terminus of the 80-kDa isoform is lost. Thus, it is possible to raise a first antibody to the N-terminal region of the 80-kDa isoform to obtain an antibody that is immunochemically reactive only with the 80-kDa isoform. A second antibody, which is immunochemically reactive with the 76-kDa, 78-kDa and 80-kDa isoforms can be used to quantitate total µCANP. Since the amount of the 78-kDa isoform does not change significantly in Alzheimer diseased brain, the ratio of the second antibody to the first antibody correlates to the ratio of the 76-kDa to 80-kDa isoforms.

Preferably, the first antibody is raised against a region in the first 21 N-terminal amino acids of µCANP. The amino acid sequence of µCANP is taught by Imajoh, S. *et al., Biochem.* 27:8122-8128 (1988). Given the amino acid sequence of µCANP, it is possible to obtain N-terminal fragments of µCANP and prepare monoclonal or polyclonal antibodies thereto. For example, one may prepare peptide segments of 8 amino acids or more by means of solid phase synthesis, conjugate the peptide segments to an immunogenic carrier, an immunize an animal therewith. Larger peptide fragments are capable of inducing, *per se,* an immune response. However, since many small molecules do not induce active immunity by themselves, it may be necessary to conjugate the peptides to an immunogenic carrier to induce production of antibodies thereto. Such immunogenic carriers include, but are not limited to, proteins such as bovine serum albumin, diphtheria toxoid, tetanus toxoid, edestin, keyhole-limpet hemocyanin, Pseudomonal Toxin A, choleragenoid, cholera toxin, pertussis toxin, viral proteins, and eukaryotic proteins such as interferons, interleukins, or tumor necrosis factor. Such proteins may be obtained from natural or recombinant sources according to methods known to those skilled in the art. When obtained from recombinant sources, the immunogenic carrier may comprise a protein fragment comprising at least the immunogenic portion of the molecule. Other known immunogenic macromolecules which may be used in the practice of the invention include, but are not limited to, polysaccharides, tRNA, nonmetabolizable synthetic polymers such as polyvinylamine, polymethacrylic acid, polyvinylpyrrolidone, mixed polycondensates (with relatively high molecular weight) of 4'4'-diaminodiphenyl-methane-3,3'-dicarboxylic acid and 4-nitro-2-aminobenzoic acid (See Sela, M., *Science 166*:1365-1374 (1969)) or glycolipids, lipids or carbohydrates. Preferably, the immunogenic carrier is a protein.

The animal immunized with the N-terminal segment of µCANP, either alone or as part of an immunogenic conjugate, may then be bled and the polyclonal sera isolated. *See* Vitto and Nixon, *J*. *Neurochem*. *47*:1039-1051 (1986). Alternatively, monoclonal antibodies may be prepared according to the well known methods of Khöler and Milstein, *Nature 256*:495-497 (1975) where the spleen of the immunized animal is removed, and the cells fused with an immortal cell line to give hybridoma cells. Hybridomas secreting the desired antibodies may be obtained by subcloning of the hybridomas in HAT selection media and screening of the resultant clones for immunogenic activity with the 80-kDa isoform and lack of immunoreactivity with the 76-kDa and 78-kDa isoforms.

Most preferably, the N-terminal segment of µCANP is the first 21 amino acids having the following amino acid sequence [SEQ ID NO. 1]:

Ala Gly Ile Ala Ala Lys Leu Ala Lys Asp Arg Glu Ala Ala Glu Gly Leu
1               5                   10                  15

Gly Ser His Glu
        20

The antibodies employed in the practice of the present invention may be detectably labelled in a manner known per se. There are many different labels and methods of labeling known to those of ordinary skill in the art. Examples of the types of labels which can be used in the present invention include, but are not limited to, enzymes, radioisotopes, dyes, fluorescent compounds, chemiluminescent compounds, bioluminescent compounds and metal chelates. Those of ordinary skill in the art will know of other suitable labels for binding to the antibodies, or will be able to ascertain the same by the use of routine experimentation. The binding of these labels to the antibodies can be accomplished using standard techniques commonly known to those of ordinary skill in the art.

One of the ways in which the antibodies can be detectably labeled is by linking the same to an enzyme. This enzyme, in turn, when later exposed to its substrate, will react with the substrate in such a manner as to produce a chemical moiety which can be detected as, for example, by spectrophotometric, fluorometric or by visual means. Enzymes which can be used to detectably label antibodies include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase.

The antibodies may also be labeled with a radioactive isotope which can be determined by such means as the use of a gamma counter or a scintillation counter or by autoradiography. Isotopes which are particularly useful for the purpose of the present invention include: ³H, ¹²⁵I, ¹³¹I, ³²P, ³⁵S, ¹⁴C or the radioisotopes of lead, mercury, thallium, technetium or indium (²⁰³Pb, ¹⁹⁸Hg, ²⁰¹Tl, ^{99m}Tc, ¹¹¹In).

It is also possible to label the antibodies with a fluorescent compound. When the fluorescently labeled annexine is exposed to light of the proper wave length, its presence can then be detected due to the fluorescence of the dye. Among the most commonly used fluorescent labelling compounds are fluorescein isothiocyanate, rhodamine, phycoerythrin, phycocyanin, allophycocyanin, o-phthaldehyde and fluorescamine.

The antibodies can also be detectably labeled using fluorescent emitting metals such as ¹⁵²Eu, or others of the lanthanide series. These metals can be attached to the antibodies using such metal chelating groups as diethylenetriaminepentaacetic acid (DTPA) or ethylenediamine-tetraacetic acid (EDTA).

Labelling is also possible by means of a paramagnetic contrast agent which is detectable in a MRI (magnetic resonance imaging) system. It is possible to use gadolinium, cobalt, nickel, manganese or iron complexes by means of which conjugates may be provided as diagnostic agents which are detectable in a MRI system. A strong magnetic field is used in such systems in order to adjust the nuclear spin vectors of the atoms in the organism. Then the field is destroyed which causes the nuclei to return to their initial state. This process is observed and recorded.

The antibodies also can be detectably labeled by coupling them to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibodies. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Another technique which may also result in greater sensitivity when used in conjunction with the present invention consists of coupling the antibody to low molecular weight haptens. The haptens can then be specifically detected by means of a second reaction. For example, it is common to use such haptens as biotin (reacting with avidin) or dinitrophenyl, pyridoxal and fluorescamine (reacting with specific anti-hapten antibodies) in this manner. Amplification strategies may be readily applied to these and other labels.

The detectable antibodies are ideally suited for the preparation of a kit. Such a kit may comprise a carrier means being compartmentalized to receive in close confinement therein one or more container means such as vials, tubes and the like, each of said container means comprising the separate elements of the assay. For example, there may be a container means containing a detectable antibody specific for all isoforms of µCANP, and further container means containing, for example, standard serial dilutions of the 76-kDa, 78-kDa and 80-kDa isoforms of µCANP in solution. The detectable antibody may be detectably labelled. A further container means may contain a means for carrying out an electrophoretic separation of the 76-kDa, 78-kDa and 80-kDa isoforms of µCANP. The standard solutions of µCANP may be used as standards for the electrophoretic separation of the µCANP isoforms of a tissue sample and to prepare standard curves with the concentration of the 76-kDa, 78-kDa and 80-kDa isoforms of µCANP plotted on the abscissa and the detection signal on the ordinate. The results obtained from the supernatant of a tissue sample such as brain cells or fibroblasts may be interpolated from such a plot to give the concentration of the 76-kDa, 78-kDa and 80-kDa isoforms of µCANP and, thus, an indication of whether the individual has Alzheimer's disease. Further container means may comprise a detectably labelled antibody that is immunoreactive with the detectable antibody that is specific for the active 76-kDa isoform of µCANP and the 80-kDa isoform of µCANP.

Alternatively, the kit may comprise a carrier means being compartmentalized to receive in close confinement therein a container means containing a first detectable antibody specific for only the 80-kDa isoform of µCANP and a second detectable antibody which is specific for all isoforms of µCANP. In a preferred embodiment, the first and second detectable antibodies are labelled with different labels which can be discriminated by different detection methods. Further container means may contain standard serial dilutions of the 76-kDa, 78-kDa and 80-kDa isoforms of µCANP in solution.

Alternatively, the kit may comprise a carrier means being compartmentalized to receive in close confinement therein a container means containing a first detectable antibody specific for only the 80-kDa isoform of µCANP and a second detectable antibody which is specific for the 76-kDa isoform of µCANP. In a preferred embodiment, the first and second detectable antibodies are labelled with different labels which can be discriminated by different detection methods. Further container means may contain standard serial dilutions of the 76-kDa and 80-kDa isoforms of µCANP in solution.

The means for separating/identifying the µCANP isoforms may include slab gels that can be used to electrophoretically separate the µCANP isoforms. Preferably, such slab gels are sodium dodecyl sulfate (SDS)-polyacrylamide gels.

In carrying out the method for detecting the isoforms of µCANP, the specific concentrations of the isoforms, the temperature and time of incubation, as well as other assay conditions may be varied, depending on various factors including the concentration of the µCANP isoforms, the nature of the sample, and the like. Those skilled in the art will be able to determine operative and optimal assay conditions by employing routine experimentation.

Detection of the detectable antibodies and, thus, the isoforms of µCANP, may be accomplished with a scintillation counter if, for example, the detectable antibodies are labelled with a radioactive gamma emitter, or by a fluorometer if the detectable label is a fluorescence emitter. In the case of an enzyme label, the detection can be accomplished by colorimetric methods which employ a substrate for the enzyme. Detection may be accomplished by comparison with the isoform standards.

Where first and second antibodies, as described herein, are employed to determine the relative ratio of the 76-kDa\80-kDa isoforms, the two antibodies may be detectably labelled with two different labels with may be separately detected. In this embodiment, the ratio may be obtained from a single incubation with both antibodies. The sample comprising the µCANP isoforms may be immobilized to a solid phase carrier such as nitrocellulose or polyvinylidene difluoride membrane (Immobilon-P), contacted with the first and second detectably labelled antibodies, and the amount of bound label determined. Other steps such as washing, stirring, shaking, filtering and the like may be added to the assay as is customary or necessary for the particular situation.

As described in more detail below, there is evidence that the activation of µCANP leads to the development of neurofibrillary pathology and β-amyloid formation which are hallmarks of Alzheimer's disease. Thus, agents which inhibit the activation of µCANP may be useful in treating or preventing the neuropathology characteristic of Alzheimer's disease. Thus, the invention also relates to a method for screening a drug for use in the treatment or prevention of Alzheimer's disease, comprising
(a) obtaining a first animal brain sample, preferably a cortical brain slice;
(b) determining the relative ratio of the active 76-kDa isoform of µCANP to the 80 kDa isoform in the first animal brain sample;
(c) incubating a second animal brain sample with an aqueous solution of Ca⁺⁺ and a drug suspected of being useful in treating or preventing Alzheimer's disease;
(d) determining the relative ratio of the active 76-kDa isoform of µCANP to the 80 kDa isoform in the tissue sample obtained in step (c);
wherein when the 76-kDa/80-kDa ratio of the animal brain sample contacted with said drug as determined in step (d) is reduced compared to the 76-kDa/80-kDa ratio obtained in step (b) confirms that the drug is useful for treating or preventing Alzheimer's disease.

Preferably, the first and second tissue samples are obtained premortem from the brain of an animal or postmortem from an individual having Alzheimer's disease.

Alternatively, the invention relates to a method for screening a drug for use in the treatment or prevention of Alzheimer's disease, comprising
(a) obtaining a first fibroblast sample from an animal;
(b) determining the relative ratio of the active 76-kDa isoform of µCANP to the 80 kDa isoform in the first fibroblast sample;
(c) incubating a second fibroblast sample with an aqueous solution of Ca⁺⁺, a calcium ionophore, and a drug suspected of being useful in treating or preventing Alzheimer's disease;
(d) determining the relative ratio of the active 76-kDa isoform of µCANP to the 80 kDa isoform in the fibroblast sample obtained in step (c);
wherein when the 76-kDa/80-kDa ratio of the fibroblast sample contacted with said drug as determined in step (d) is reduced compared to the 76-kDa/80-kDa ratio obtained in step (b) confirms that the drug is useful for treating or preventing Alzheimer's disease.

In step (b) of either method, the tissue sample may be first homogenized in a buffer and centrifuged to give a supernatant and then the relative ratio of the active 76-kDa isoform of µCANP to the 80-kDa isoform in the supernatant is then determined. The relative ratio of the active 76-kDa isoform of µCANP to the 80-kDa isoform may be determined by
(e) separating the isoforms of µCANP in the supernatant by gel electrophoresis,
(f) contacting the separated 76-kDa isoform of µCANP and the 80-kDa isoform of µCANP with at least one detectable antibody which is immunoreactive therewith,
(g) detecting the amount of detectable antibody bound to the 76-kDa and the 80-kDa isoforms of µCANP to give a relative signal for each isoform, and
(h) dividing the relative signal corresponding to the 76-kDa isoform by the relative signal corresponding to the 80-kDa isoform to give said ratio.

Alternatively, the relative ratio of the active 76-kDa isoform of µCANP to the 80-kDa isoform may be determined by
(e) contacting the supernatant with a first and a second detectable antibody, wherein the first detectable antibody is immunoreactive only with the 80-kDa isoform of µCANP and the second detectable antibody is immunoreactive with the 76-kDa, 78-kDa and 80-kDa isoforms of µCANP;
(f) detecting the amount of detectable first and second antibodies bound to the respective isoforms of µCANP to give a relative signal; and
(g) dividing the relative signal corresponding to the 76-kDa, 78-kDa and 80-kDa isoforms by the relative signal corresponding to the 80-kDa isoform to give said ratio.

The conversion of the 80-kDa isoform to the 76-kDa isoform is calcium dependent. Thus, in step (c), the tissue sample is first incubated with calcium and the drug for a time sufficient to alter the 76-kDa/80-kDa ratio. Typically, the drug and calcium are contacted with the tissue sample for about 5 to about 60 min. The concentration of calcium may range from 0-5 mM. The concentration of the drug may range from 0.001 to about 1000 µM. Preferably, the tissue sample is first incubated with the drug at about 4°C and is then incubated at about 30 °C with calcium and the drug. When fibroblasts are employed, a calcium ionophore such as A23187 is employed at a concentration range of 1-100 µM. The tissue sample is then homogenized in a buffer and centrifuged to give a supernatant, and then the relative ratio of the active 76-kDa isoform of µCANP to the 80-kDa isoform in said supernatant is determined.

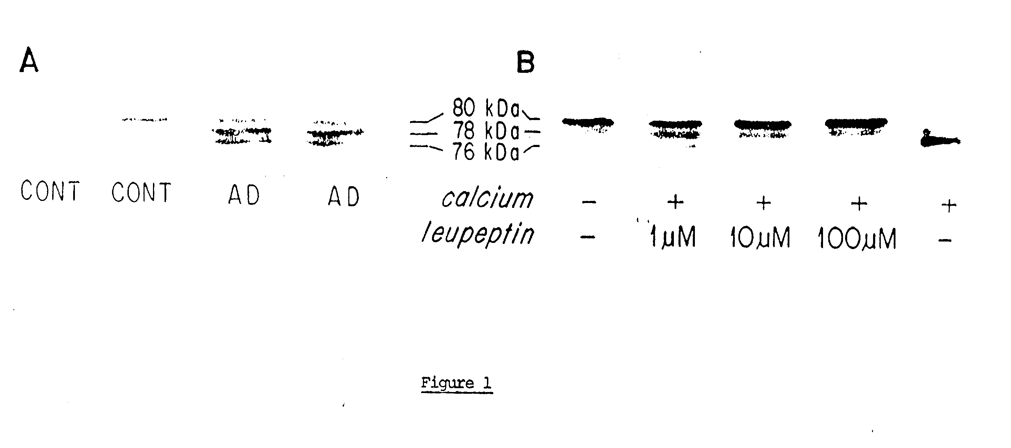

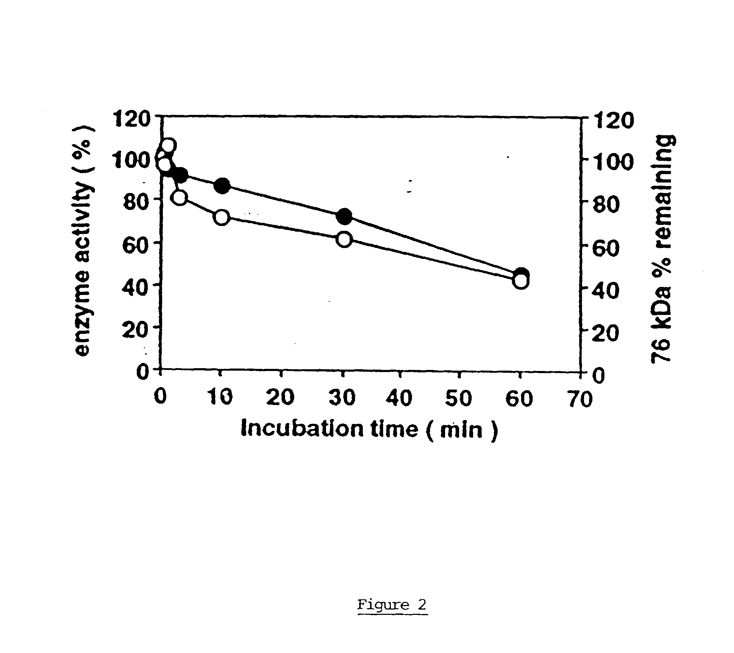

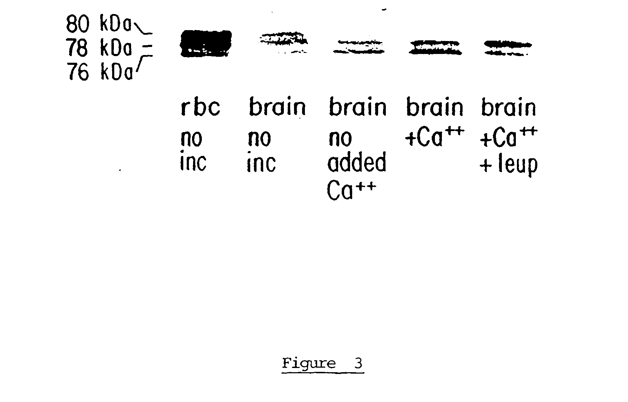

In the practice of the invention, a monoclonal antibody raised against human µCANP (Samis, J.A., *et al., Biochem*. *J. 246:*481-488 (1987); and Elce, J.S., *et al., Biochem*. *J. 261*:1039-1042 (1989) may be used to detect by Western blot analysis the three isoforms of the catalytic subunit of µCANP in human brain extracts. These isoenzymes exhibit molecular masses on SDS polyacrylamide gels of 80 kDa, 78 kDa and 76 kDa identical to those in human erythrocytes (Samis, J.A., *et al., Biochem*. *J. 246:*481-488 (1987); Elce, J.S., *et al., Biochem. J. 261*:1039-1042 (1989); Samis *et al., Biochem. J. 246*:481-487 (1987)) (Fig. 1a). When human erythrocyte µCANP was activated *in vitro* by adding calcium, hydrolysis of purified spectrin by the enzyme was accompanied by N-terminal cleavage of the 80-kDa isoform at the GLY₂₆-LEU₂₇ peptide bond and formation of the 76-kDa isoform (The 76-kDa isoform of µCANP, generated by autolysis, separated by SDS-PAGE and transferred to DF-immobilon membranes, was subjected to N-terminal analysis by the Edman degradation method at the Harvard University Microchemistry Facility, courtesy of William Lane.) (Fig. lb). Conversion of the 80-kDa µCANP isoform to 78-kDa and 76-kDa isoforms was nearly complete within 30 sec. Enzyme activity was preserved in the absence of 80-kDa µCANP and remained proportional to the amount of 76-kDa isoform, confirming other recent data that the 76-kDa isoform is enzymatically active (Pontremoli, S., *et al., Biochem. Biophys. Res. Commun. 123(1):*331-337 (1984); Inomata, M., *et al., Biochem. Biophys. Res. Commun. 138(2)*:638-643 (1986)) (Fig. 2). Although human brain µCANP is unstable in cell free extracts, conversion of the 80-kDa isoform of µCANP to the 76-kDa isoform could be demonstrated in slices of human neo-cortex incubated with calcium (Fig. 3). Autolysis of µCANP was confirmed by demonstrating loss of the extreme N-terminus of the 80-kDa isoform using a polyclonal antibody raised against the first 21 amino acids of the polypeptide (A 34-mer synthetic peptide corresponding to the extreme N-terminal amino acid sequence of µCANP deduced from a human liver cDNA (Imajoh *et al., Biochem. 27:* 8122 (1988)) was used unconjugated to immunize 2-month-old New Zealand rabbits as described previously (Vitto & Nixon, *J. Neurochem. 47*:1039-1051 (1986)). The amino acid sequence of this 34-mer is as follows [SEQ. ID NO. 2]:

Ser Glu Glu Ile Ile Thr Pro Val Tyr Cys Thr Gly Val Ser Ala Gln Val Gln
1               5                   10                  15

Lys Gln Arg Ala Arg Glu Leu Gly Leu Gly Arg His Glu Asn Ala Ile
    20                  25                  30

Antiserum prepared against this 34-mer specifically cross-reacted with the 80-kDa isoform of µCANP on Western blot analyses of 30,000 xg supernatant extract from human brain.

To evaluate the relative state of µCANP activation in the brain in Alzheimer disease, the three µCANP isoforms were quantitated by immunoassay of extracts of brain tissue from 22 individuals who had premortem diagnoses of dementia and met neuropathological criteria for Alzheimer disease, and 17 individuals who were free of clinical neurologic disease (Khachaturian, Z.S., *Arch. Neurol. 42*:1097-1105 (1985)) and neuropathological evidence (Mirra, S.S., *et al., Neurology 41*:479-486 (1991)) for Alzheimer disease. The Alzheimer and control groups were matched with respect to age of the individual (75.8 ± 2.0 years vs. 68.9 ± 2.6 years, respectively), and postmortem interval for brain samples (12.2 ± 1.3 hr vs. 12.0 ± 1.8 hr, respectively).

The content of cytosolic 80-kDa µCANP was substantially lower in the Alzheimer group than in controls (22.7 ± 1.5% vs. 37.2 ± 2.2%, p < 0.001, unpaired t-test). By contrast, autolytically activated (76 kDa) µCANP was increased nearly two-fold in Alzheimer brain (41.2 ± 1.6%) than in controls (26.6 ± 2.2%) (p < 0.001). The 78-kDa µCANP isoform, was unchanged. The ratio of 76-kDa to 80-kDa µCANP for individual brain samples was nearly 3-fold higher in the Alzheimer group (2.20 ± 0.39 vs. 0.81 ± 0.10). The sum of the three µCANP isoforms was not changed (Table 1).

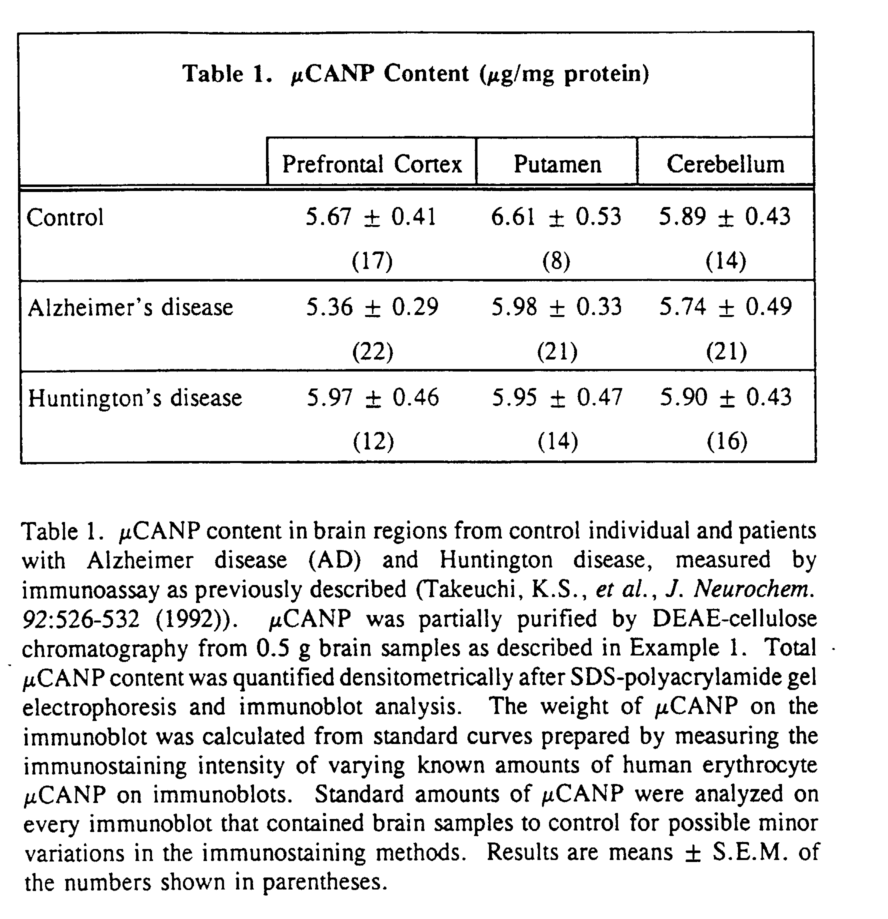

The abnormal ratio of µCANP isoforms in the cytosolic fraction did not reflect a shift in their cellular distribution (Table 2). About 30% of the total µCANP was associated with the particulate fraction in both control and Alzheimer groups (Table 2). The higher proportion of 76-kDa µCANP observed in this fraction is consistent with the hypothesis that µCANP is activated mainly on the membrane (Inomata, M., *et al*., *J*. *Biol*. *Chem*. *264(31)*:18838-18843 (1969); Pontremoli, S., *et al*., *Biochem*. *Biophys*. *Res*. *Commun*. *128(1)*:331-338 (1985)). Interestingly, the 76-kDa to 80-kDa isoform ratios in the cytosolic fractions from control brains correlated significantly with those in corresponding particulate fractions of control brains (r = 0.839, p < 0.005), but this relationship was disturbed in Alzheimer brain (r = 0.077).

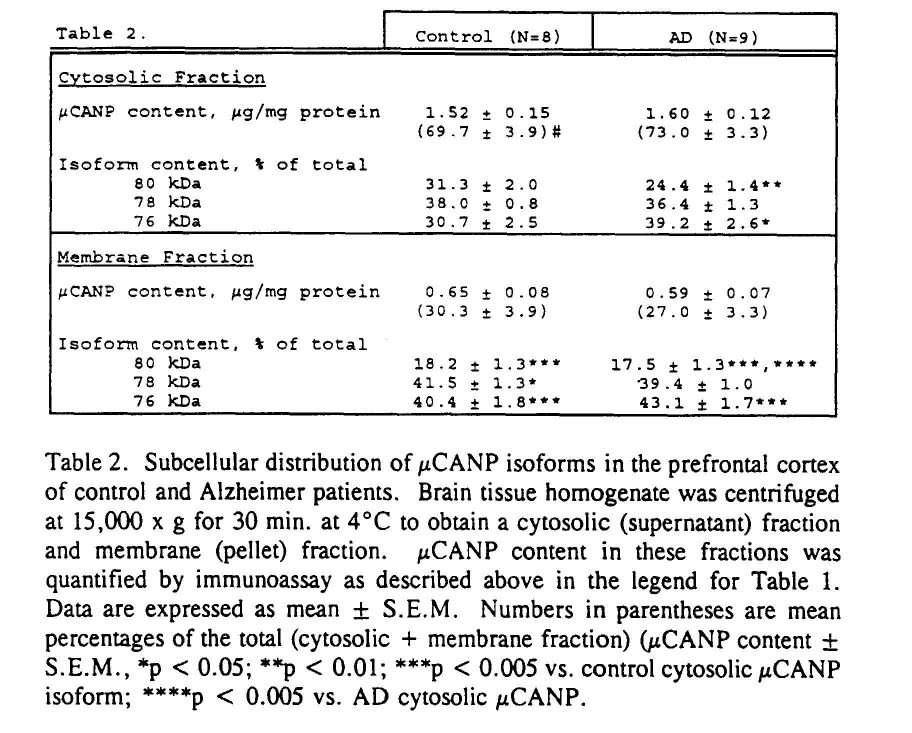

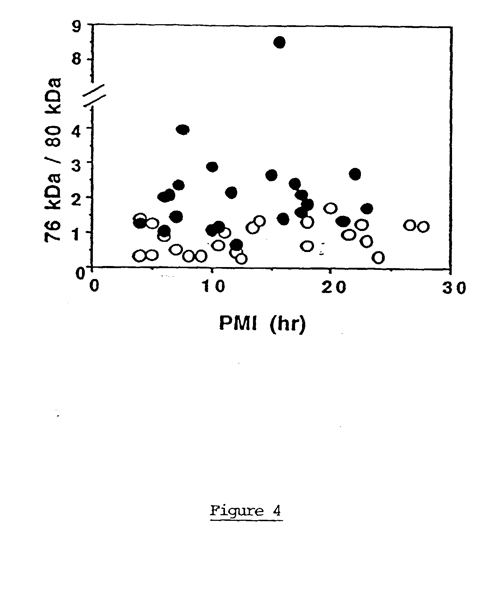

The degree of abnormal µCANP activation was comparable in Alzheimer brains at all postmortem intervals. The ratio of 76 kDa to 80 kDa isoforms regressed against postmortem interval yielded non-significant correlations in control (r = 0.355), Alzheimer (r = 0.155), or combined groups (r = 0.148) (Fig. 4). The possibility cannot be excluded, however, that isoform ratios change significantly within the first few hours after death. The degree of µCANP activation also did not significantly correlate with age within the range studied for any group.

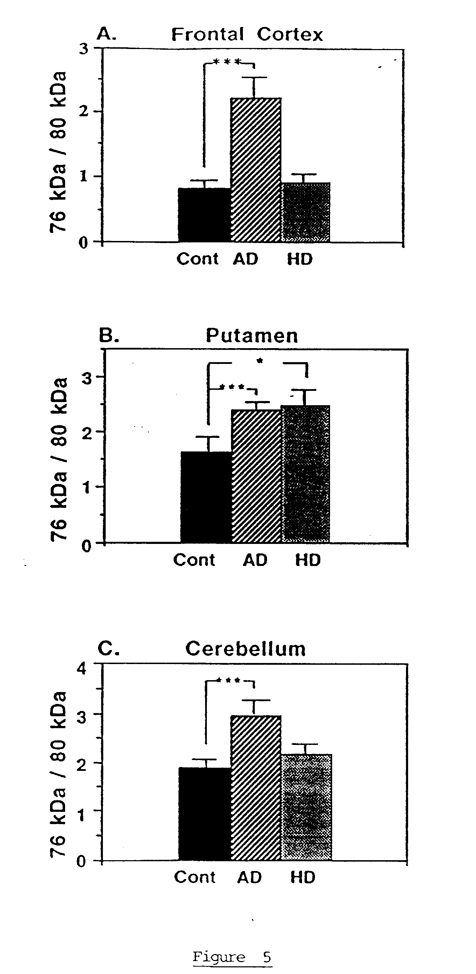

Because some neurons are degenerating in Alzheimer neocortex, the degree of µCANP activation in two brain areas was examined where neuronal degeneration is reported to be minimal or absent (Pro, J.D., *et al*., *Neurology* *30*:820-825 (1980); Katzman & Terry, in *The Neurobiology of of Aging,* Katzman & Terry, eds., F.A. Davis Co., New York, pp. 51-84 (1983)). The ratio of 76-kDa to 80-kDa µCANP isoforms was significantly elevated in both putamen (46%, p < 0.005) and cerebellum (58%, p < 0.005) in the Alzheimer cases (Fig. 5b,c). By contrast, the proportions of µCANP isoforms were normal in the prefrontal cortex and cerebellum from patients with Stage III Huntington disease (Fig. 5A and C). Consistent with evidence that ongoing neuronal degeneration is associated with CANP activation (Dure, L.S., *et al*., *Ann*. *Neurol*. *30*:785-793 (1991)), µCANP activation was increased 50% (p < 0.05) in samples of putamen from Huntington patients (HD) (Vonsattel, J.-P., *et al*., *J*. *Neuropathol*. *Exp*. *Neurol*. *44*:559-577 (1985); Roos, R.A.C., *et al*., *J*. *Neurol*. *Neurosurg*. *Psychiatry 48*:422-425 (1985)), where neuronal cell loss is considerable (Fig. 5b).

The modestly increased µCANP activation seen in Huntington putamen could possibly be explained by influx of calcium associated with either end-stage cell death occurring in this region or NMDA receptor stimulation, which is believed to be an underlying mechanism of neuronal cell death in Huntington's disease (Dure, L.S., *et al*., *Ann*. *Neurol*. *30*:785-793 (1991)). The much higher levels of µCANP activation in Alzheimer neocortex and the similar abnormalities in brain regions exhibiting minimal neuronal degeneration indicate that µCANP activation is not a consequence simply of end-stage neuronal degeneration but reflects a more widespread metabolic alteration that precedes and possibly contributes to neuronal degeneration. In this regard, the extensive loss of synapses in neocortex, which correlates with dementia (Terry *et al*., *Ann*. *Neurol*. *30*:572-580 (1991); Hamos *et al*., *Ann*. *Neurol*. *39*: 355 (1991); Dekosky & Scheff, *Ann*. *Neurol*. *27*:457-464 (1990)) is relevant since µCANP is enriched at synapses (Peterson, C., *et al*., *Neurosci*. *Lett. 121*:239-243 (1991)) and activation of CANP at nerve terminals has been associated with terminal degeneration (Swanson & Vrbóva, *Devel*. *Brain Res*. *33*:199-203 (1987); O'Brien, R.A.D., *et al*., *Neuroscience 12*:637-646 (1984)).

Widespread µCANP activation in brain is consistent with evidence in fibroblasts of Alzheimer patients for increased degradation of spectrin (Peterson, C., *et al*., *Neurosci*. *Lett*. *121*:239-243 (1991)) and diminished content of protein kinase C (Saitoh, T., *et al*., *Lab*. *Invest*. *64*:596-616 (1991)) which are two preferred substrates of CANPs. Brain spectrin breakdown is also increased in cortex and hippocampus of Alzheimer brain (Masliah, E., *et al*., *Brain Res*. *531*:36-44 (1990)). Previous reports that total CANP activity is not significantly altered in Alzheimer brain (Kawashima, S., *et al*., *Biomed. Res*. *10(1)*:17-23 (1989); Mantle & Perry, *J*. *Neurol*. *Sci*. *102*:220-224 (1991); Nilsson, E., *et al*., *Neurobiol*. *Aging 11*:425-431 (1990)) accord with our results and suggest that measurements of the total CANP pool may not sensitively reflect functional activity of the CANP system.

Apart from contributing to synaptic loss and cell death, abnormal µCANP activation may have additional consequences in neurons that bear on the development of neurofibrillary pathology and β-amyloid formation. These include the abnormal processing of cytoskeletal proteins and other sensitive CANP substrates, which include the amyloid precursor protein (APP) (Siman *et al*., in *Neurotoxicity of Excitatory Amino Acids*, A. Guidotti, Ed. (Raven Press, New York, 1990), pp. 145-161; Lee *et al*., *Proc*. *Natl*. *Acad*. *Sci USA 88*:7233-7347 (1991) and alterations of the activities of calcium-dependent protein kinases and phosphatases (Togari, A., *et al.*, *Biochem*. *Biophys*. *Res*. *Commun*. *134(2)*:749-754 (1968); Kishimoto, A., *et al*., *J*. *Biol*. *Chem*. *258*:1156-1164 (1983); Tallant, E.A., *et al*., *Biochem*. *27*:2205-2211 (1988); Nixon, R.A., *Ann*. *N*.*Y*. *Acad*. *Sci*. *568*:198-208 (1989)) which may in turn also affect APP processing or secretion. In this regard, reduced levels of soluble (secreted) forms of APP have been found in the cerebrospinal fluid of Alzheimer patients. In preliminary studies, the degree of µCANP activation in the neocortex of Alzheimer patients exhibited a highly significant inverse correlation with the level of soluble APP in human brain. These results are compatible with the hypothesis that β-amyloid or APP fragments have a primary role in Alzheimer-related neurodegeneration. The toxicity of β-amyloid amyloid or APP fragments may, in part, be mediated through alterations in calcium homeostasis, which would be expected to lead to CANP activation.

Pharmacological modulation of the CANP system, which is effective *in vivo* in limiting neural damage in ischemia and vasospasm (Siman, R., in *Neurotoxicity of Excitatory Amino Acids,* A. Guidotti, ed., Raven Press, New York, pp. 145-161 (1990); Lee, K.S., *et al., Proc. Natl. Acad. Sci. USA* 88:7233-7347 (1991); Minami, N., *et al., J. Neurosurg.* 76: 111-118(1992)), merits further consideration as a potential therapeutic strategy in Alzheimer disease.

### Examples

### Example 1 Immunoblot Analysis of µCANP in Prefrontal Cortex

Fig. 1(A) depicts the immunoblot analysis of µCANP in prefrontal cortex from two control (CONT) and two Alzheimer (AD) patients. Prefrontal cortex (0.5 g) from control or AD patients was homogenized with 10 vol. of 20 mM Tris HCI, pH 7.4, containing 2 mM EGTA, 1 mM EDTA, 1 mM benzamidine, 1 mM dithiothreitol, 1 mM phenylmethylsulfonyl fluoride (PMSF) and 0.32 M sucrose (Buffer A). The homogenate was centrifuged at 15,000 x g for 30 min at 4°C and the supernatant was applied to a column (1.0 x 15 cm) of DEAE-cellulose (DE-52, Whatman®) equilibrated with Buffer A. The column, extensively washed with Buffer A, was eluted with stepwise increments of KCI from 0 to 3 mM. µCANP was eluted from the column by 0.15 M KCI in Buffer A. These fractions were subjected to 7.5% SDS-PAGE according to the method of Laemmli, *Nature 227*:680-685 (1970). Electroblotting was performed according to Towbin *et al., Proc. Natl*. *Acad.* Sci. *USA 76:4350-4354* (1979), in a transfer buffer containing 15% methanol, 25 m*M* Tris and 192 m*M* glycine, pH 8.3. Proteins were transferred Electrophetically onto PVDF-membrane (Immobilon-P, Millipore®) by applying 0.5 A of constant current for 2.5 h at 4°C. The blots were incubated with 5 % nonfat dry milk solution containing 2 m*M* EGTA, 0.15 *M* NaCl in 50 m*M* Tris-HCl, pH 7.4 (blocking buffer) for 1 h at room temperature. Immunoblot analysis was carried out with a rabbit monoclonal anti-µCANP antibody using peroxidase-conjugated goat anti-rabbit immunoglobulin G for 5 h at 4°C. After several washes of the blots with 50 m*M* Tris-HCl, pH 7.5, 0.15 *M* NaCl, peroxidase activity was visualized with H₂O₂, 4-chloro-1-naphthol and N, N'-dimethyl-p-phenylenediamine. Figure 1(A) shows µCANP in prefrontal cortex of two representative control brains (lanes 1, 2) and two AD brains (lanes 3, 4).

### Example 2 Immunoblot Analysis of the Autolysis Products of µCANP

Fig. 1(B) depicts the immunoblot analysis of the autolysis products of µCANP from human erythrocytes *in vitro.* Human erythrocyte µCANP was purified from normal adult blood as described before (Inomata, M., *et al., J. Biochem.* 93:291-294 (1983); Fairbanks, G., *et al., Biochemistry 10(13)*:2606 (1971)) (lane 1); incubation with 5.3 mM CaCl₂ (final free calcium concentration was 50 µM) and 2.2 µg of human erythrocyte spectrin (Bennet, V., *et al., Nature* 299:126 (1982)) for 30 sec at 30°C in the presence of either 1 µM leupeptin (lane 2), 10 µM leupeptin (lane 3), or 100 µM leupeptin (lane 4), or incubation with 5.3 mM CaCl₂ and 2.2 µg of spectrin for 30 sec at 30°C (lane 5).

Fig. 2 shows the relationship between enzyme activity and µCANP content. After preincubation of human erythrocyte µCANP with calcium and spectrin, 1 mM EGTA was added to stop the reaction. Half of each sample was applied to SDS/PAGE and immunoreactivity of each µCANP isoform was quantified using a Bio-Rad Video Densitometer Model 620. The other half was used to measure enzyme activity as previously described (Takeuchi, K.S., *et al*., *J*. *Neurochem*. *92*:526-532 (1992)) by adding [¹⁴C]azocacein as substrate and 4 mM CaCl₂ (final free calcium concentration was 125 µM). Note that the 78 kDa isoform completely disappears by 1 min of incubation. O: protease activity. ●: µCANP 76 kDa content.

Fig. 3 shows µCANP autolysis in human brain slices. Sections of fresh postmortem human brain (prefrontal cortex) (200 µM) were incubated at 37°C for 60 min with Buffer A with or without CaCl₂ (final concentration 5 mM). Slices were then rinsed with Buffer A (1 ml x 2), homogenized, and centrifuged 15,000 x g for 30 min at 4°C. The supernatant was subjected to immunoblot analysis as described above. Human erythrocyte µCANP (lane 1); human brain µCANP from unincubated slices (lane 2) or slices incubated in the absence of exogenous calcium (lane 3), 5 mM CaCl₂ (lane 4), or with 5 mM CaCl₂ plus 10 µM leupeptin (lane 5).

### Example 3 Comparison of Control with Alzheimer's Diseased Brains

Fig. 4 depicts a graph showing the ratio of 76 kDa/80 kDa isoforms of µCANP in prefrontal cortex from control individuals (O) and AD patients (●), plotted against postmortem interval (PMI). Immunoassay of individual isoforms (Fig. 1) was performed as described in the legend to Table 1.

### Example 4 Comparison of Isoform Levels in Alzheimer's Disease Brain and Huntington's Disease Brain

Fig. 5 depicts the µCANP isoform ratios (76 kDa/80 kDa) in brain regions from control, Alzheimer and Huntington disease (HD) brains measured by Western blot immunoassay as described in Example 3. (A) Prefrontal cortex: mean values ± S.E.M. for PMI and age from HD (n=12), control (n=17), AD cases (n=22) were 10.5 ± 1.9 hr and 54.6 ± 3.3 years. ****p < 0.005. (B) Putamen: The mean values ± S.E.M. of PMI and age were 10.6 ± 2.9 hr and 68.5 ± 1.9 years, 14.8 ± 1.4 hr and 71.8 ± 2.3 years, or 14.1 ± 2.0 hr and 60.1 ± 2.6 years in control (n=8), AD (n=21) or HD (n=14) patients, respectively. *p < 0.05, ***p < 0.05. (C) Cerebellum: The mean ± S.E.M. of PMI and age were 7.6 ± 1.6 hr and 67.8 ± 4.2 years, 13.1 ± 1.7 hr and 76.0 ± 1.9 years, or 10.6 ± 2.1 hr and 54.6 ± 3.1 years in control (n=14), AD (n=21) or HD (n=16) patients, respectively. ***p < 0.005.

### Example 5 Abnormalities in calpain regulation in Alzheimer's disease --Fibroblast studies

### Methods

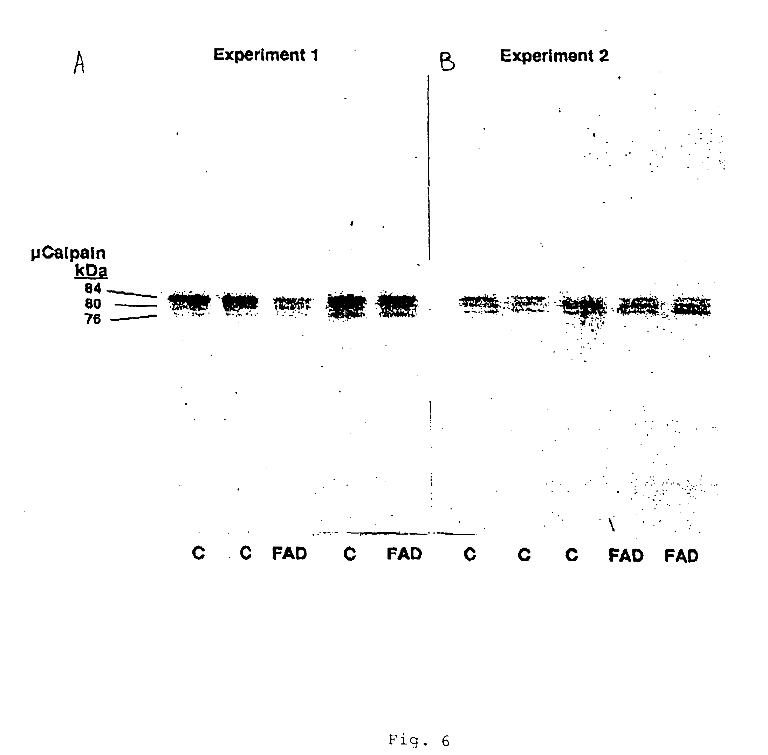

Experiments using fibroblasts from FAD cohorts were carried out in collaboration with Dr. Jesse Sisken (University of Kentucky Medical School), who provided fibroblast samples from control (C) and AD-affected individuals. Stocks were maintained in DMEM (Gibco) supplemented with 15% FBS (Sigma). Cells were set up on Corning 100-mm culture plates in DMEM (Gibco) supplemented with 17% FBS (Gibco). After 1-2 days, the cultures were considered "subconfluent" and were harvested. The cells were gently scraped off the surface with a sterile rubber policeman. The cells were then transferred to a centrifuge tube, centrifuged, and the cell pellet was resuspended in medium with FBS, transferred to a cryovial, recentrifuged and frozen. Alternatively, cells were harvested by addition of 0.05% trypsin-EDTA, neutralization by medium with FBS. Immunoassay of the individual isoforms was performed as described in the legend to Table 1. The results are depicted in Fig. 6 (panels A and B).

### Results

In collaboration with Dr. Jesse Sisken (University of Kentucky Medical School), studies of the calpain system were initiated in cultured fibroblasts from individuals from a large familial Alzheimer disease cohort, in which Alzheimer disease is expressed early and in which the gene defect has been localized to chromosome 21 (but is not at the APP locus) (Nee *et al*., Arch. *Neurol*. *40*:203-208 (1984); Tanzi *et al*., *Science 235*:880-884 (1987); Tanzi *et al*., *Nature 329*:156-157 (1987); Tanzi *et al*., *Nature 331*:528-530 (1988)). Studies to date on 7 controls and 6 phenotypically affected individuals (two experiments are shown in Fig. 6), have identified an abnormally high ratio of the activated µcalpain isoform (76 kDa) to the latent (80 kDa) µcalpain isoform in the AD cases (p < 0.02, paired t test). The demonstration of the same type of µcalpain abnormality in fibroblasts as seen in postmortem human brain, supports the idea that this abnormality is widespread in AD and is not related solely to end-stage cell death phenomena. Thus, fibroblast cells are useful models for investigating calpain system activation in relation to downstream effects on kinases, APP metabolism and other AD-related processes.

## Claims

1. A method for the detection of Alzheimer's disease in an individual, comprising
(a) obtaining a tissue sample from an individual suspected of having Alzheimer's disease; and
(b) determining the relative ratio of the active 76-kDa isoform of µCANP to the 80-kDa isoform in said tissue sample;
wherein an increased ratio of 76-kDa/80-kDa compared to a control group of individuals not having Alzheimer's disease confirms that the individual suspected of having Alzheimer's disease does have Alzheimer's disease.

2. The method of claim 1, wherein said tissue sample is obtained from the brain of said individual.

3. The method of claim 1, wherein said tissue sample is obtained from the frontal cortex of said individual.

4. The method of claim 1, wherein said tissue sample is a fibroblast tissue sample obtained from said individual.

5. The method of claim 1, wherein in step (b), said tissue sample is first homogenized in a buffer and centrifuged to give a supernatant and then the relative ratio of the active 76-kDa isoform of µCANP to the 80-kDa isoform in said supernatant is determined.

6. The method of claim 5, wherein the relative ratio of the active 76-kDa isoform of µCANP to the 80-kDa isoform is determined by
(c) separating the isoforms of µCANP by gel electrophoresis,
(d) contacting the separated 76-kDa isoform of µCANP and the 80-kDa isoform of µCANP with at least one detectable antibody which is immunoreactive therewith,
(e) detecting the amount of detectable antibody bound to the 76-kDa and the 80-kDa isoforms of µCANP to give a relative signal for each isoform, and
(e) dividing the relative signal corresponding to the 76-kDa isoform by the relative signal corresponding to the 80-kDa isoform to give said ratio.

7. The method of claim 6, wherein said detectable antibody is detectably labelled and the amount of detectably labelled antibody is determined by detecting the label.

8. The method of claim 6, wherein said detectable antibody is detected by contacting said detectable antibody with a second, detectably labelled antibody which is immunoreactive with said detectable antibody.

9. A method for the detection of Alzheimer's disease in an individual, comprising
(a) obtaining a tissue sample from an individual suspected of having Alzheimer's disease; and
(b) determining the relative ratio of the active 76-kDa isoform of µCANP to the total quantities of the 76-kDa, 78-kDa and 80-kDa isoforms; wherein an increased ratio of 76-kDa/76-kDa+78-KDa+80-kDa compared to a control group of individuals not having Alzheimer's disease confirms that the individual suspected of having Alzheimer's disease has Alzheimer's disease.

10. The method of claim 9, wherein said tissue sample is obtained from the brain of said individual.

11. The method of claim 9, wherein said tissue sample is obtained from the frontal cortex of said individual.

12. The method of claim 9, wherein said tissue sample is a fibroblast tissue sample obtained from said individual.

13. The method of claim 9, wherein in step (b), said tissue sample is first homogenized in a buffer and centrifuged to give a supernatant and then the relative ratio of the active 76-kDa isoform of µCANP to the 76-kDa, 78-kDa and 80-kDa isoforms in said supernatant is determined.

14. The method of claim 13, wherein the relative ratio of the active 76-kDa isoform of µCANP to the 76-kDa, 78-kDa and 80-kDa isoforms is determined by
(c) contacting the supernatant with a first and a second detectable antibody, wherein the first detectable antibody is immunoreactive only with the 80-kDa isoform of µCANP and the second detectable antibody is immunoreactive with the 76-kDa, 78-kDa and 80-kDa isoforms of µCANP;
(d) detecting the amount of detectable first and second antibodies bound to the respective isoforms of µCANP to give a relative signal; and
(e) dividing the relative signal corresponding to the 76-kDa, 78-kDa and 80-kDa isoforms by the relative signal corresponding to the 80-kDa isoform to give said ratio.

15. The method of claim 14, wherein said first and second detectable antibodies are detectably labelled and the amount of detectably labelled antibodies is determined by detecting the respective labels.

16. The method of claim 14, wherein said first and second detectable antibodies are detected by contacting them with at least one detectably labelled antibody which is immunoreactive with said first and second detectable antibodies, and detecting the label.

17. A kit which is useful for detecting Alzheimer's disease in a individual suspected of having the disease, comprising a carrier means having in close confinement therein one or more container means, wherein a first container means contains a detectable antibody that is immunoreactive with the active 76-kDa isoform of µCANP and the 80-kDa isoform of µCANP.

18. The kit of claim 17, wherein said detectable antibody is detectably labelled.

19. The kit of claim 17, further comprising a second container means containing a detectably labelled antibody that is immunoreactive with said detectable antibody that is immunoreactive with the active 76-kDa isoform of µCANP and the 80-kDa isoform of µCANP.

20. The kit of claim 17, further comprising a third container means which contains a means for separating the 76-kDa isoform of µCANP and the 80-kDa isoform of µCANP.

21. A kit which is useful for detecting Alzheimer's disease in a individual suspected of having the disease, comprising a carrier means having in close confinement therein two or more container means, wherein a first container means contains a first detectable antibody that is immunoreactive with the 80-kDa isoform of µCANP and is not immunoreactive with the 76-kDa and 78-kDa isoforms of µCANP and a second container means contains a second detectable antibody that is immunoreactive with the 76-kDa, 78-kDa and 80-kDa isoforms of µCANP.

22. The kit of claim 21, wherein said first and second detectable antibodies are detectably labelled with different labels that can be discriminated on detection.

23. A kit which is useful for detecting Alzheimer's disease in a individual suspected of having the disease, comprising a carrier means being compartmentalized to receive in close confinement therein a container means containing a first detectable antibody specific for only the 80-kDa isoform of µCANP and a second detectable antibody which is specific only for the 76-kDa isoform of µCANP.

24. The kit of claim 23, wherein the first and second detectable antibodies are labelled with different labels which can be discriminated by different detection methods.

25. The kit of claim 23, further comprising a container means which contains standard serial dilutions of the 76-kDa and 80-kDa isoforms of µCANP in solution.

26. A method for screening a drug for use in the treatment or prevention of Alzheimer's disease, comprising
(a) obtaining a first animal brain sample;
(b) determining the relative ratio of the active 76-kDa isoform of µCANP to the 80 kDa isoform in the first animal brain sample;
(c) incubating a second animal brain sample with an aqueous solution of Ca⁺⁺ and a drug suspected of being useful in treating or preventing Alzheimer's disease;
(d) determining the relative ratio of the active 76-kDa isoform of µCANP to the 80 kDa isoform in the tissue sample obtained in step (c); wherein when the 76-kDa/80-kDa ratio of the animal brain sample contacted with said drug as determined in step (d) is reduced compared to the 76-kDa/80-kDa ratio obtained in step (b) confirms that the drug is useful for treating or preventing Alzheimer's disease.

27. The method of claim 26, wherein said first and second brain samples are cortical brain slices obtained immediately postmortem from the brain of an animal.

28. The method of claim 26, wherein said first and second brain samples are cortical brain slices obtained postmortem from an individual having Alzheimer's disease.

29. The method of claim 26, wherein in step (b), said brain sample is first homogenized in a buffer and centrifuged to give a supernatant and then the relative ratio of the active 76-kDa isoform of µCANP to the 80-kDa isoform in said supernatant is determined.

30. The method of claim 29, wherein the relative ratio of the active 76-kDa isoform of µCANP to the 80-kDa isoform is determined by
(e) separating the isoforms of µCANP in said supernatant by gel electrophoresis,
(f) contacting the separated 76-kDa isoform of µCANP and the 80-kDa isoform of µCANP with at least one detectable antibody which is immunoreactive therewith,
(g) detecting the amount of detectable antibody bound to the 76-kDa and the 80-kDa isoforms of µCANP to give a relative signal for each isoform, and
(h) dividing the relative signal corresponding to the 76-kDa isoform by the relative signal corresponding to the 80-kDa isoform to give said ratio.

31. The method of claim 30, wherein said detectable antibody is detectably labelled and the amount of detectable antibody is determined in step (g) by detecting the label.

32. The method of claim 30, wherein said detectable antibody is detected in step (g) by contacting said detectable antibody with a second, detectably labelled antibody which is immunoreactive with said detectable antibody and detecting the label.

33. The method of claim 29, wherein the relative ratio of the active 76-kDa isoform of µCANP to the 80-kDa isoform is determined by
(e) contacting said supernatant with a first and a second detectable antibody, wherein the first detectable antibody is immunoreactive only with the 80-kDa isoform of µCANP and the second detectable antibody is immunoreactive with the 76-kDa, 78-kDa and 80-kDa isoforms of µCANP;
(f) detecting the amount of detectable first and second antibodies bound to the respective isoforms of µCANP to give a relative signal; and
(g) dividing the relative signal corresponding to the 76-kDa, 78-kDa and 80-kDa isoforms by the relative signal corresponding to the 80-kDa isoform to give said ratio.

34. The method of claim 33, wherein said detectable antibody is detectably labelled and the amount of detectable antibody is determined in step (f) by detecting the label.

35. The method of claim 33, wherein said first and second detectable antibodies are detectably labelled with two different labels.

36. A method for screening a drug for use in the treatment or prevention of Alzheimer's disease, comprising
(a) obtaining a first fibroblast sample from an animal;
(b) determining the relative ratio of the active 76-kDa isoform of µCANP to the 80 kDa isoform in the first fibroblast sample;
(c) incubating a second fibroblast sample with an aqueous solution of Ca⁺⁺, a calcium ionophore, and a drug suspected of being useful in treating or preventing Alzheimer's disease;
(d) determining the relative ratio of the active 76-kDa isoform of µCANP to the 80 kDa isoform in the fibroblast sample obtained in step (c);
wherein when the 76-kDa/80-kDa ratio of the fibroblast sample contacted with said drug as determined in step (d) is reduced compared to the 76-kDa/80-kDa ratio obtained in step (b) confirms that the drug is useful for treating or preventing Alzheimer's disease.

37. The method of claim 36, wherein in step (b), said fibroblast sample is first homogenized in a buffer and centrifuged to give a supernatant and then the relative ratio of the active 76-kDa isoform of µCANP to the 80-kDa isoform in said supernatant is determined.

38. The method of claim 37, wherein the relative ratio of the active 76-kDa isoform of µCANP to the 80-kDa isoform is determined by
(e) separating the isoforms of µCANP in said supernatant by gel electrophoresis,
(f) contacting the separated 76-kDa isoform of µCANP and the 80-kDa isoform of µCANP with at least one detectable antibody which is immunoreactive therewith,
(g) detecting the amount of detectable antibody bound to the 76-kDa and the 80-kDa isoforms of µCANP to give a relative signal for each isoform, and
(h) dividing the relative signal corresponding to the 76-kDa isoform by the relative signal corresponding to the 80-kDa isoform to give said ratio.

39. The method of claim 38, wherein said detectable antibody is detectably labelled and the amount of detectable antibody is determined in step (g) by detecting the label.

40. The method of claim 38, wherein said detectable antibody is detected in step (g) by contacting said detectable antibody with a second, detectably labelled antibody which is immunoreactive with said detectable antibody and detecting the label.

41. The method of claim 37, wherein the relative ratio of the active 76-kDa isoform of µCANP to the 80-kDa isoform is determined by
(e) contacting said supernatant with a first and a second detectable antibody, wherein the first detectable antibody is immunoreactive only with the 80-kDa isoform of µCANP and the second detectable antibody is immunoreactive with the 76-kDa, 78-kDa and 80-kDa isoforms of µCANP;
(f) detecting the amount of detectable first and second antibodies bound to the respective isoforms of µCANP to give a relative signal; and
(g) dividing the relative signal corresponding to the 76-kDa, 78-kDa and 80-kDa isoforms by the relative signal corresponding to the 80-kDa isoform to give said ratio.

42. The method of claim 41, wherein said detectable antibody is detectably labelled and the amount of detectable antibody is determined in step (f) by detecting the label.

43. The method of claim 41, wherein said first and second detectable antibodies are detectably labelled with two different labels.

## Patentansprüche

1. Verfahren zur Feststellung der Alzheimer-Krankheit in einem Individuum, umfassend
(a) Erhalten einer Gewebeprobe von einem Individuum, von dem angenommen wird, daß es die Alzheimer-Krankheit hat, und
(b) Bestimmen des relativen Verhältnisses der aktiven 76-kDa µCANP-Isoform zu der 80-kDA Isoform in der Gewebeprobe, wobei ein erhöhtes 76-kDa/80-kDA Verhältnis im Vergleich zu einer Kontrollgruppe von Individuen, die nicht die Alzheimer-Krankheit haben, bestätigt, daß das Individuum, von dem angenommen wird, daß es die Alzheimer-Krankheit hat, die Alzheimer-Krankheit hat.

2. Verfahren nach Anspruch 1, worin die Gewebeprobe von dem Gehirn des Individuums erhalten wird.

3. Verfahren nach Anspruch 1, worin die Gewebeprobe von der Stirnlappenkortex des Individuums erhalten wird.

4. Verfahren nach Anspruch 1, worin die Gewebeprobe eine von dem Individuum erhaltene Fibroblasten-Gewebeprobe ist.

5. Verfahren nach Anspruch 1, worin in Schritt (b) die Gewebeprobe zunächst unter Erhalt eines Überstandes in einem Puffer homogenisiert und zentrifugiert wird und anschließend das relative Verhältnis der aktiven 76-kDa µCANP-Isoform zu der 80-kDA Isoform in dem Überstand bestimmt wird.

6. Verfahren nach Anspruch 5, worin das relative Verhältnis der aktiven 76-kDa µCANP-Isoform zu der 80-kDA Isoform bestimmt wird durch
(c) Trennen der µCANP-Isoformen durch Gelelektrophorese,
(d) Kontaktieren der getrennten 76-kDa µCANP-Isoform und der 80-kDA µCANP-Isoform mit mindestens einem nachweisbaren Antikörper, der mit diesen immunoreaktiv ist,
(e) Feststellen der Menge des nachweisbaren an die 76-kDa und die 80-kDA µCANP-Isoformen gebundenen Antikörpers, um ein relatives Signal für jede Isoform zu erhalten, und
(e) Dividieren des relativen Signals entsprechend der 76-kDa Isoform durch das relative Signal entsprechend der 80-kDA Isoform, um das Verhältnis zu erhalten.

7. Verfahren nach Anspruch 6, worin der nachweisbare Antikörper feststellbar markiert wird, und die Menge des nachweisbar markierten Antikörpers durch Feststellen der Markierung bestimmt wird.

8. Verfahren nach Anspruch 6, worin der nachweisbare Antikörper durch Kontaktieren des nachweisbaren Antikörpers mit einem zweiten nachweisbar markierten Antikörper, der mit diesem nachweisbaren Antikörper immunoreaktiv ist, festgestellt wird.

9. Verfahren zur Feststellung der Alzheimer-Krankheit in einem Individuum, umfassend
(a) Erhalten einer Gewebeprobe von einem Individuum, von dem angenommen wird, daß es die Alzheimer-Krankheit hat, und
(b) Bestimmen des relativen Verhältnisses der aktiven 76-kDa µCANP-Isoform zu den Gesamtmengen der 76-kDA, 78-kDA und 80-kDA Isoformen, wobei ein erhöhtes Verhältnis von 76-kDa/76-kDa + 78-kDa ± 80-kDA im Vergleich zu einer Kontrollgruppe von Individuen, die nicht die Alzheimer-Krankheit haben, bestätigt, daß das Individuum, von dem angenommen wird, daß es die Alzheimer-Krankheit hat, die Alzheimer-Krankheit hat.

10. Verfahren nach Anspruch 9, worin die Gewebeprobe von dem Gehirn des Individuums erhalten wird.

11. Verfahren nach Anspruch 9, worin die Gewebeprobe von der Stirnlappenkortex des Individuums erhalten wird.

12. Verfahren nach Anspruch 9, worin die Gewebeprobe eine von dem Individuum erhaltene Fibroblasten-Gewebeprobe ist.

13. Verfahren nach Anspruch 9, worin in Schritt (b) die Gewebeprobe zunächst unter Erhalt eines Überstandes in einem Puffer homogenisiert und zentrifugiert wird und anschließend das relative Verhältnis der aktiven 76-kDa µCANP-Isoform zu den 76-kDA, 78-kDA und 80-kDA Isoformen in dem Überstand bestimmt wird.

14. Verfahren nach Anspruch 13, worin das relative Verhältnis der aktiven 76-kDA µCANP-Isoform zu den 76-kDA, 78-kDA und 80-kDA Isoformen bestimmt wird durch
(c) Kontaktieren des Überstandes mit einem ersten und einem zweiten nachweisbar markierten Antikörper, wobei der erste nachweisbare Antikörper nur mit der 80-kDA µCANP-Isoform und der zweite nachweisbare Antikörper mit den 76-kDA, 78-kDA und 80-kDA µCANP-Isoformen immunoreaktiv ist,
(d) Feststellen der Menge des nachweisbaren ersten und zweiten Antikörpers, der an die jeweiligen µCANP-Isoformen bindet, um ein relatives Signal zu erhalten, und
(e) Dividieren des relativen Signals entsprechend den 76-kDA, 78-kDA und 80-kDA Isoformen durch das relative Signal entsprechend der 80-kDA Isoform, um das Verhältnis zu erhalten.

15. Verfahren nach Anspruch 14, worin der erste und zweite nachweisbare Antikörper feststellbar markiert werden, und die Menge der nachweisbar markierten Antikörper durch Feststellen der jeweiligen Markierungen bestimmt wird.

16. Verfahren nach Anspruch 14, worin der erste und zweite nachweisbare Antikörper festgestellt werden durch Kontaktieren derselben mit mindestens einem nachweisbar markierten Antikörper, der mit dem ersten und zweiten nachweisbaren Antikörper immunoreaktiv ist, und Feststellen der Markierung,.

17. Kit, das zur Feststellung der Alzheimer-Krankheit in einem Individuum, von dem angenommen wird, daß es die Alzheimer-Krankheit hat, nützlich ist, umfassend eine Trägereinrichtung, die einen oder mehrere Behältereinrichtungen mit enger Begrenzung hat, worin der erste Behälter einen nachweisbaren Antikörper enthält, der mit der aktiven 76-kDa µCANP-Isoform und der 80-kDa µCANP-Isoform immunoreaktiv ist.

18. Kit nach Anspruch 17, worin der nachweisbare Antikörper feststellbar markiert ist.

19. Kit nach Anspruch 17, außerdem umfassend einen zweiten Behälter, der einen nachweisbaren Antikörper enthält, der mit dem nachweisbaren Antikörper immunoreaktiv ist, der mit der aktiven 76-kDa µCANP-Isoform und der 80-kDa µCANP-Isoform immunoreaktiv ist.

20. Kit nach Anspruch 17, außerdem umfassend einen dritten Behälter, der ein Mittel zum Trennen der 76-kDa µCANP-Isoform und der 80-kDa µCANP-Isoform enthält.

21. Kit, das zur Feststellung der Alzheimer-Krankheit in einem Individuum, von dem angenommen wird, daß es die Alzheimer-Krankheit hat, nützlich ist, umfassend eine Trägereinrichtung, die zwei oder mehrere Behältereinrichtungen mit enger Begrenzung hat, worin der erste Behälter einen ersten nachweisbaren Antikörper enthält, der mit der 80-kDa µCANP-Isoform und nicht mit den 76-kDa und 80-kDa µCANP-Isoformen immunoreaktiv ist, und ein zweiter Behälter einen zweiten nachweisbaren Antikörper enthält, der mit den 76-kDa, 78-kDa und 80-kDa µCANP-Isoformen immunoreaktiv ist.

22. Kit nach Anspruch 21, worin der erste und zweite nachweisbare Antikörper mit verschiedenen Markierungen, die beim Nachweis unterschieden werden können, nachweisbar markiert sind.

23. Kit, das zur Feststellung der Alzheimer-Krankheit in einem Individuum, von dem angenommen wird, daß es die Alzheimer-Krankheit hat, nützlich ist, umfassend eine unterteilte Trägereinrichtung, die mit enger Begrenzung eine Behältereinrichtung enthält, die einen ersten nachweisbaren Antikörper, der nur für die 80-kDa µCANP-Isoform spezifisch ist und einen zweiten nachweisbaren Antikörper, der nur für die 76-kDa µCANP-Isoform spezifisch ist, enthält.

24. Kit nach Anspruch 23, worin der erste und zweite nachweisbare Antikörper mit verschiedenen Markierungen, die beim Nachweis unterschieden werden können, nachweisbar markiert sind.

25. Kit nach Anspruch 23, außerdem umfassend einen Behälter, der Standard-Serienverdünnungen der 76-kDa und 80-kDa µCANP-Isoformen in Lösung enthält.

26. Verfahren zum Screenen eines Medikaments zur Verwendung bei der Behandlung oder Vorbeugung der Alzheimer-Krankheit, umfassend
(a) Erhalten einer ersten Tierhirnprobe,
(b) Bestimmen des relativen Verhältnisses der aktiven 76-kDa µCANP-Isoform zu der 80-kDa Isoform in der ersten Tierhirnprobe,
(c) Inkubieren einer zweiten Tierhirnprobe mit einer wäßrigen Lösung aus Ca⁺⁺ und einem Medikament, von dem angenommen wird, daß es beim Behandeln oder Vorbeugen der Alzheimer-Krankheit nützlich ist;
(d) Bestimmen des relativen Verhältnisses der aktiven 76-kDa µCANP-Isoform zu der 80-kDa Isoform in der in Schritt (c) erhaltenen Gewebeprobe,
wobei bestätigt wird, daß das Medikament zum Behandeln oder Vorbeugen der Alzheimer-Krankheit nützlich ist, wenn sich das 76-kDa/80-kDA Verhältnis der Tierhirnprobe, die mit dem Medikament kontaktiert wird, wie in Schritt (d) bestimmt, im Vergleich zu dem in Schritt (b) erhaltenen 76-kDa/80-kDA Verhältnis verringert.

27. Verfahren nach 26, worin die erste und zweite Hirnprobe Corticohirnscheiben sind, die post-mortem von dem Tierhirn unmittelbar erhalten werden.

28. Verfahren nach 26, worin die erste und zweite Hirnprobe Corticohirnscheiben sind, die von einem Individuum mit Alzheimer-Krankheit post-mortem erhalten werden.

29. Verfahren nach Anspruch 26, worin in Schritt (b) die Hirnprobe zunächst unter Erhalt eines Überstandes in einem Puffer homogenisiert und zentrifugiert wird und anschließend das relative Verhältnis der aktiven 76-kDa µCANP-Isoform zu der 80-kDa Isoform in dem Überstand bestimmt wird.

30. Verfahren nach Anspruch 29, worin das relative Verhältnis der aktiven 76-kDa µCANP-Isoform zu der 80-kDa Isoform bestimmt wird durch
(e) Trennen der µCANP-Isoformen in dem Überstand durch Gelelektrophorese,
(f) Kontaktieren der getrennten 76-kDa µCANP-Isoform und der 80-kDA µCANP-Isoform mit mindestens einem nachweisbaren Antikörper, der mit diesen immunoreaktiv ist,
(g) Feststellen der Menge des nachweisbaren Antikörpers, der an die 76-kDa und die 80-kDA µCANP-Isoformen gebunden ist, um ein relatives Signal für jede Isoform zu erhalten, und
(h) Dividieren des relativen Signals entsprechend der 76-kDa Isoform durch das relative Signal entsprechend der 80-kDA Isoform, um das Verhältnis zu erhalten.

31. Verfahren nach Anspruch 30, worin der nachweisbare Antikörper feststellbar markiert wird, und die Menge des nachweisbaren Antikörpers in Schritt (g) durch Feststellen der Markierung bestimmt wird.

32. Verfahren nach Anspruch 30, worin der nachweisbare Antikörper in Schritt (g) festgestellt wird durch Kontaktieren des nachweisbaren Antikörpers mit einem zweiten nachweisbar markierten Antikörper, der mit dem nachweisbaren Antikörper immunoreaktiv ist, und Feststellen der Markierung.

33. Verfahren nach Anspruch 29, worin das relative Verhältnis der aktiven 76-kDa µCANP-Isoform zu der 80-kDa Isoform bestimmt wird durch
(e) Kontaktieren des Überstandes mit einem ersten und einem zweiten nachweisbar markierten Antikörper, wobei der erste nachweisbare Antikörper nur mit der 80-kDA µCANP-Isoform und der zweite nachweisbare Antikörper mit den 76-kDA, 78-kDA und 80-kDA µCANP-Isoformen immunoreaktiv ist,
(f) Feststellen der Menge des nachweisbaren ersten und zweiten Antikörpers, der an die jeweiligen µCANP-Isoformen bindet, um ein relatives Signal zu erhalten, und
(g) Dividieren des relativen Signals entsprechend den 76-kDA, 78-kDA und 80-kDA Isoformen durch das relative Signal entsprechend der 80-kDA Isoform, um das Verhältnis zu erhalten.

34. Verfahren nach Anspruch 33, worin der nachweisbare Antikörper feststellbar markiert wird, und die Menge des nachweisbaren Antikörper in Schritt (f) durch Feststellen der Markierung bestimmt wird.

35. Verfahren nach Anspruch 33, worin der erste und zweite nachweisbare Antikörper mit zwei verschiedenen Markierungen feststellbar markiert werden.

36. Verfahren zum Screenen eines Medikaments zur Verwendung bei der Behandlung oder Vorbeugung der Alzheimer-Krankheit, umfassend
(a) Erhalten einer ersten Fibroblastenprobe von einem Tier,
(b) Bestimmen des relativen Verhältnisses der aktiven 76-kDa µCANP-Isoform zu der 80-kDa Isoform in der ersten Fibroblastenprobe,
(c) Inkubieren einer zweiten Fibroblastenprobe mit einer wäßrigen Lösung aus Ca⁺⁺, einem Calciumionophor und einem Medikament, von dem angenommen wird, daß es beim Behandeln oder Vorbeugen der Alzheimer-Krankheit nützlich ist;
(d) Bestimmen des relativen Verhältnisses der aktiven 76-kDa µCANP-Isoform zu der 80-kDa Isoform in der in Schritt (c) erhaltenen Fibroblastenprobe,
wobei bestätigt wird, daß das Medikament zum Behandeln oder Vorbeugen der Alzheimer-Krankheit nützlich ist, wenn sich das 76-kDa/80-kDA Verhältnis der Fibroblastenprobe, die mit dem Medikament kontaktiert wird, wie in Schritt (d) bestimmt, im Vergleich zu dem in Schritt (b) erhaltenen 76-kDa/80-kDA Verhältnis verringert.

37. Verfahren nach Anspruch 36, worin in Schritt (b) die Fibroblastenprobe zunächst unter Erhalt eines Überstandes in einem Puffer homogenisiert und zentrifugiert wird und anschließend das relative Verhältnis der aktiven 76-kDa µCANP-Isoform zu der 80-kDa Isoform in dem Überstand bestimmt wird.

38. Verfahren nach Anspruch 37, worin das relative Verhältnis der aktiven 76-kDa µCANP-Isoform zu der 80-kDa Isoform bestimmt wird durch
(e) Trennen der µCANP-Isoformen in dem Überstand durch Gelelektrophorese,
(f) Kontaktieren der getrennten 76-kDa µCANP-Isoform und der 80-kDA µCANP-Isoform mit mindestens einem nachweisbaren Antikörper, der mit diesen immunoreaktiv ist,
(g) Feststellen der Menge des nachweisbaren Antikörpers, der an die 76-kDa und 80-kDA µCANP-Isoformen gebunden ist, unter Erhalt eines relativen Signals für jede Isoform, und
(h) Dividieren des relativen Signals entsprechend der 76-kDa Isoform durch das relative Signal entsprechend der 80-kDA Isoform, um das Verhältnis zu erhalten.

39. Verfahren nach Anspruch 38, worin der nachweisbare Antikörper feststellbar markiert ist, und die Menge des nachweisbar markierten Antikörpers in Schritt (g) durch Feststellen der Markierung bestimmt wird.

40. Verfahren nach Anspruch 38, worin der nachweisbare Antikörper in Schritt (g) festgestellt wird durch Kontaktieren des nachweisbaren Antikörpers mit einem zweiten nachweisbar markierten Antikörper, der mit dem nachweisbaren Antikörper immunoreaktiv ist, und Feststellen der Markierung.

41. Verfahren nach Anspruch 37, worin das relative Verhältnis der aktiven 76-kDa µCANP-Isoform zu der 80-kDa Isoform bestimmt wird durch
(e) Kontaktieren des Überstandes mit einem ersten und einem zweiten nachweisbaren Antikörper, wobei der erste nachweisbare Antikörper nur mit der 80-kDA µCANP-Isoform und der zweite nachweisbare Antikörper mit den 76-kDA, 78-kDA und 80-kDA µCANP-Isoformen immunoreaktiv ist,
(f) Feststellen der Menge des nachweisbaren ersten und zweiten Antikörpers, der an die jeweiligen µCANP-Isoformen gebunden ist, um ein relatives Signal zu erhalten, und
(g) Dividieren des relativen Signals entsprechend den 76-kDA, 78-kDA und 80-kDA Isoformen durch das relative Signal entsprechend der 80-kDA Isoform, um das Verhältnis zu erhalten.

42. Verfahren nach Anspruch 41, worin der nachweisbare Antikörper feststellbar markiert wird, und die Menge des nachweisbar markierten Antikörpers in Schritt (f) durch Feststellen der Markierung bestimmt wird.

43. Verfahren nach Anspruch 41, worin der erste und zweite nachweisbare Antikörper mit zwei verschiedenen Markierungen feststellbar markiert werden.

## Revendications

1. Méthode pour le dépistage de la maladie d'Alzheimer chez un individu, comprenant les étapes consistant :
(a) à obtenir un échantillon de tissu d'un individu chez qui on suspecte la maladie d'Alzheimer; et
(b) à déterminer le rapport relatif de l'isoforme active de 76 kDa sur l'isoforme de 80 kDa de la µ-protéinase neutre
activée par le calcium (µ-PNAC) dans ledit échantillon de tissu; dans laquelle une augmentation du rapport isoforme de 76 kDa/isoforme de 80 kDa, par comparaison avec celui d'un groupe de contrôle d'individus qui ne sont pas atteints de la maladie d'Alzheimer, confirme que l'individu chez qui on suspecte la maladie d'Alzheimer en est atteint.

2. Méthode selon la revendication 1, dans laquelle ledit échantillon de tissu est obtenu du cerveau dudit individu.

3. Méthode selon la revendication 1, dans laquelle ledit échantillon de tissu est obtenu à partir du cortex frontal dudit individu.

4. Méthode selon la revendication 1, dans laquelle ledit échantillon de tissu est un échantillon de tissu fibroblastique obtenu dudit individu.

5. Méthode selon la revendication 1 dans laquelle, à l'étape (b), ledit échantillon de tissu est d'abord homogénéisé dans un tampon et centrifugé pour obtenir un surnageant, dans lequel est ensuite déterminé le rapport relatif de l'isoforme active de 76 kDa sur l'isoforme de 80 kDa de la protéinase µ-PNAC.

6. Méthode selon la revendication 5, dans laquelle le rapport relatif de l'isoforme active de 76 kDa sur l'isoforme de 80 kDa de la protéinase µ-PNAC est déterminé :
(c) en séparant les isoformes de la protéinase µ-PNAC par électrophorèse sur gel,
(d) en mettant en contact les isoformes séparées de 76 kDa et de 80 kDa de la protéinase µ-PNAC avec au moins un anticorps détectable qui est immunoréactif avec celles-ci,
(e) en détectant la quantité d'anticorps détectable lié à l'isoforme de 76 kDa et à l'isoforme de 80 kDa de la protéinase µ-PNAC pour donner un signal relatif pour chaque isoforme et
(f) en divisant le signal relatif correspondant à l'isoforme de 76 kDa par le signal relatif correspondant à l'isoforme 80 kDa pour obtenir ledit rapport.

7. Méthode selon la revendication 6, dans laquelle ledit anticorps détectable est marqué de manière détectable et la quantité d'anticorps marqué de manière détectable est déterminée en détectant le marqueur.

8. Méthode selon la revendication 6, dans laquelle ledit anticorps détectable est détecté en mettant en contact ledit anticorps détectable avec un second anticorps marqué de manière détectable, qui est immunoréactif avec ledit anticorps détectable.

9. Méthode pour le dépistage de la maladie d'Alzheimer chez un individu comprenant les étapes consistant
(a) à obtenir un échantillon de tissu d'un individu chez qui on suspecte la maladie Alzheimer; et
(b) à déterminer le rapport relatif de l'isoforme active de 76 kDa sur la quantité totale des isoformes de 76 kDa, 78 kDa et de 80 kDa de la protéinase µ-PNAC;
dans laquelle un rapport augmenté isoforme de 76 kDa/isoforme de 76 kDa + isoforme de 78 kDa + isoforme de 80 kDa, comparé à celui d'un groupe de contrôle d'individus qui ne sont pas atteints de la maladie d'Alzheimer, confirme que l'individu chez qui on suspecte la maladie Alzheimer en est atteint.

10. Méthode selon la revendication 9, dans laquelle ledit échantillon de tissu est obtenu à partir du cerveau dudit individu.

11. Méthode selon la revendication 9, dans laquelle ledit échantillon de tissu est obtenu du cortex frontal dudit individu.

12. Méthode selon la revendication 9, dans laquelle ledit échantillon de tissu est un échantillon de tissu fibroblastique obtenu dudit individu.

13. Méthode selon la revendication 9, dans laquelle, à l'étape (b), ledit échantillon de tissu est d'abord homogénéisé dans un tampon et centrifugé pour donner un surnageant et ensuite, le rapport relatif de l'isoforme active de 76 kDa sur le total des isoformes 76 kDa, 78 kDa et 80 kDa de la protéinase µ-PNAC est déterminé dans ledit surnageant.

14. Méthode selon la revendication 13, dans laquelle le rapport relatif de l'isoforme active de 76 kDa sur le total des isoformes de 76 kDa, 78 kDa et 80 kDa de la protéinase µ-PNAC est déterminé
(c) en mettant en contact le surnageant avec un premier et un second anticorps détectables, le premier anticorps détectable étant immunoréactif uniquement avec l'isoforme de 80 kDa de la protéinase µ-PNAC et le second anticorps détectable étant immunoréactif avec les isoformes de 76 kDa, de 78 kDa et de 80 kDa de la protéinase µ-PNAC;
(d) en détectant la quantité des premier et second anticorps détectables liés aux isoformes respectives de la protéinase µ-PNAC pour donner un signal relatif; et
(e) en divisant le signal relatif correspondant aux isoformes de 76 kDa, 78 kDa et 80 kDa par le signal relatif correspondant à l'isoforme de 80 kDa pour obtenir ledit rapport.

15. Méthode selon la revendication 14, dans laquelle lesdits premier et second anticorps détectables sont marqués de manière détectable et la quantité d'anticorps marqués de manière détectable est déterminée en détectant les marqueurs respectifs.

16. Méthode selon la revendication 14, dans laquelle lesdits premier et second anticorps détectables sont détectés en les mettant en contact avec au moins un anticorps marqué de manière détectable, qui est immunoréactif avec ledit premier et ledit second anticorps détectables et en détectant le marqueur.

17. Coffret utile pour le dépistage de la maladie d'Alzheimer chez un individu chez qui on suspecte la maladie, comprenant un réceptacle pour loger contenant un ou plusieurs récipients étroitement groupés, dans lequel un premier récipient contient un anticorps détectable qui est immunoréactif avec l'isoforme active de 76 kDa de la protéinase µ-PNAC et l'isoforme de 80 kDa de la protéinase µ-PNAC.

18. Coffret selon la revendication 17, dans lequel ledit anticorps détectable est marqué de manière détectable.

19. Coffret selon la revendication 17, comprenant en outre un second réceptacle contenant un anticorps marqué de manière détectable qui est immunoréactif avec ledit anticorps détectable qui est immunoréactif avec l'isoforme active de 76 kDa de la protéinase µ-PNAC et l'isoforme 80 kDa de la protéinase µ-PNAC.

20. Coffret selon la revendication 17, comprenant en outre un troisième réceptacle contenant un moyen pour séparer l'isoforme de 76 kDa de la protéinase µ-PNAC et l'isoforme de 80 kDa de la protéinase µ-PNAC.

21. Coffret utile pour le dépistage de la maladie d'Alzheimer chez un individu chez qui on suspecte cette maladie, comprenant un réceptacle contenant deux ou plusieurs récipients étroitement groupés, dans lequel un premier récipient contient un premier anticorps détectable qui est immunoréactif avec l'isoforme de 80 kDa de la protéinase µ-PNAC et qui n'est pas immunoréactif avec les isoformes de 76 kDa et de 78 kDa de la protéinase µ-PNAC et un second récipient qui contient un second anticorps détectable qui est immunoréactif avec les isoformes de 76 kDa, de 78 kDa et de 80 kDa de la protéinase µ-PNAC.

22. Coffret selon la revendication 21, dans lequel les premier et second anticorps détectables sont marqués de manière détectable avec différents marqueurs qui peuvent être distingués à la détection.

23. Coffret utile pour le dépistage de la maladie d'Alzheimer chez un individu que l'on suspecte en être atteint, comprenant un réceptacle réalisé avec des compartiments pour recevoir, étroitement groupés, un récipient contenant un premier anticorps détectable spécifique uniquement vis-à-vis de l'isoforme de 80 kDa de la protéinase µ-PNAC et un second anticorps spécifique uniquement vis-à-vis de l'isoforme de 76 kDa de la protéinase µ-PNAC.

24. Coffret selon la revendication 23, dans lequel lesdits premier et second anticorps détectables sont marqués avec différents marqueurs qui peuvent être distingués par différentes méthodes.

25. Coffret selon la revendication 23, comprenant en outre un récipient contenant des dilutions standard de solutions des isoformes de 76 kDa et de 80 kDa de la protéinase µ-PNAC.

26. Méthode pour évaluer un médicament destiné au traitement ou à la prévention de la maladie d'Alzheimer comprenant les étapes consistant :
(a) à obtenir un premier échantillon du cerveau d'un animal;
(b) à déterminer le rapport relatif de l'isoforme active de 76 kDa sur l'isoforme de 80 kDa de la protéinase µ-PNAC dans le premier échantillon du cerveau de l'animal;
(c) à incuber un second échantillon du cerveau de l'animal dans une solution aqueuse de Ca⁺⁺ et d'un médicament présumé utile pour le traitement ou la prévention de la maladie d'Alzheimer; et
(d) à déterminer le rapport relatif de l'isoforme active de 76 kDa sur l'isoforme de 80 kDa de la protéinase µ-PNAC dans l'échantillon de tissu obtenu à l'étape (c);
dans laquelle une diminution du rapport isoforme de 76 kDa /isoforme de 80 kDa de l'échantillon du cerveau de l'animal mis en contact avec ledit médicament déterminé dans l'étape (d) par comparaison avec le rapport isoforme de 76 kDa/isoforme de 80 kDa obtenu à l'étape (b) confirme que le médicament est utile pour traiter la maladie d'Alzheimer.

27. Méthode selon la revendication 26, dans laquelle les premier et second échantillons du cerveau sont des tranches du cortex cérébral obtenues immédiatement post mortem du cerveau d'un animal.

28. Méthode selon la revendication 26, dans laquelle les premier et second échantillons du cerveau sont des tranches de cortex cérébral obtenues post mortem d'un individu atteint de la maladie d'Alzheimer.

29. Méthode selon la revendication 26, dans laquelle à l'étape (b), ledit échantillon de cerveau est d'abord homogénéisé dans un tampon et centrifugé pour obtenir un surnageant dans lequel est déterminé le rapport relatif de l'isoforme active de 76 kDa sur l'isoforme de 80 kDa de la protéinase µ-PNAC.

30. Méthode selon la revendication 29, dans laquelle le rapport relatif de l'isoforme active de 76 kDa sur l'isoforme de 80 kDa de la protéinase µ-PNAC est déterminé
(e) en séparant les isoformes de la protéinase µ-PNAC dans ledit surnageant par électrophorèse sur gel;
(f) en mettant en contact les isoformes séparées de 76 kDa et de 80 kDa de la protéinase µ-PNAC avec au moins un anticorps détectable qui est immunoréactif avec elles;
(g) en détectant la quantité d'anticorps détectable lié aux isoformes de 76 kDa et de 80 kDa de la protéinase µ-PNAC pour obtenir un signal relatif pour chaque isoforme et
(h) en divisant le signal relatif correspondant à l'isoforme de 76 kDa par le signal relatif correspondant à l'isoforme de 80 kDa pour obtenir ledit rapport.

31. Méthode selon la revendication 30, dans laquelle ledit anticorps détectable est marqué de manière détectable et la quantité d'anticorps détectable est déterminée à l'étape (g) par la détection du marqueur.

32. Méthode selon la revendication 30, dans laquelle ledit anticorps détectable est détecté à l'étape (g) en mettant en contact ledit anticorps détectable avec un second anticorps marqué de manière détectable, qui est immunoréactif avec ledit anticorps détectable et en détectant ledit marqueur.

33. Méthode selon la revendication 29, dans laquelle le rapport relatif de l'isoforme active de 76 kDa sur l'isoforme de 80 kDa de la protéinase µ-PNAC est déterminé :
(e) en mettant en contact ledit surnageant avec un premier et un second anticorps détectables, le premier anticorps détectable étant immunoréactif uniquement avec l'isoforme de 80 kDa de la protéinase µ-PNAC et le second anticorps détectable étant immunoréactif avec les isoformes de 76 kDa, 78 kDa et 80 kDa de la protéinase µ-PNAC;
(f) en détectant la quantité des premier et second anticorps liés aux isoformes respectives de la protéinase µ-PNAC pour obtenir un signal relatif; et
(g) en divisant le signal relatif correspondant aux isoformes de 76 kDa, 78 kDa et 80 kDa par le signal relatif correspondant à l'isoforme de 80 kDa, pour obtenir ledit rapport.

34. Méthode selon la revendication 33, dans laquelle ledit anticorps détectable est marqué de manière détectable et la quantité d'anticorps détectable est déterminée à l'étape (f) en détectant le marqueur.

35. Méthode selon la revendication 33, dans laquelle lesdits premier et second anticorps détectables sont marqués de manière détectable avec deux marqueurs différents.

36. Méthode pour évaluer un médicament pour le traitement ou la prévention de la maladie d'Alzheimer, comprenant les étapes consistant :
(a) à obtenir un premier échantillon fibroblastique d'un animal;
(b) à déterminer le rapport relatif de l'isoforme active de 76 kDa sur l'isoforme de 80 kDa de la protéinase µ-PNAC dans le premier échantillon fibroblastique;
(c) à incuber un second échantillon fibroblastique avec une solution aqueuse de Ca²⁺, un ionophore du calcium et un médicament présumé utile pour le traitement ou la prévention de la maladie d'Alzheimer; et
(d) à déterminer le rapport relatif de l'isoforme active de 76 kDa sur l'isoforme de 80 kDa de la protéinase µ-PNAC dans l'échantillon fibroblastique obtenu à l'étape (c);
dans laquelle une diminution du rapport isoforme de 76 kDa/isoforme de 80 kDa de l'échantillon fibroblastique en contact avec ledit médicament comme déterminé à l'étape (d), par comparaison avec le rapport isoforme de 76 kDa/isoforme de 80 kDa obtenu à l'étape (b) confirme que le médicament est utile pour traiter ou prévenir la maladie d'Alzheimer.

37. Méthode selon la revendication 36, dans laquelle, à l'étape (b), ledit échantillon fibroblastique est d'abord homogénéisé dans un tampon et centrifugé pour donner un surnageant, dans lequel est déterminé ensuite le rapport relatif de l'isoforme active de 76 kDa sur l'isoforme de 80 kDa de la protéinase µ-PNAC.

38. Méthode selon la revendication 37, dans laquelle le rapport relatif de l'isoforme active de 76 kDa sur l'isoforme de 80 kDa de la protéinase µ-PNAC est déterminé :
(e) en séparant les isoformes de la protéinase µ-PNAC dans ledit surnageant par électrophorèse sur gel,
(f) en mettant en contact les isoformes séparées de 76 kDa et de 80 kDa de la protéinase µ-PNAC avec au moins un anticorps détectable qui est immunoréactif avec elles,
(g) en détectant la quantité d'anticorps détectable lié à l'isoforme de 76 kDa et à l'isoforme de 80 kDa de la protéinase µ-PNAC pour donner un signal relatif pour chaque isoforme et
(h) en divisant le signal relatif correspondant à l'isoforme de 76 kDa par le signal relatif correspondant à l'isoforme de 80 kDa, pour obtenir ledit rapport.

39. Méthode selon la revendication 38, dans laquelle ledit anticorps détectable est marqué de manière détectable et la quantité d'anticorps détectable est déterminée dans l'étape (g) en détectant le marqueur.

40. Méthode selon la revendication 38, dans laquelle ledit anticorps détectable est détecté à l'étape (g) en mettant en contact ledit anticorps détectable avec un second anticorps marqué de manière détectable qui est immunoréactif avec ledit anticorps détectable et en détectant le marqueur.

41. Méthode selon la revendication 37, dans laquelle le rapport relatif de l'isoforme active de 76 kDa sur l'isoforme de 80 kDa de la protéinase µ-PNAC est déterminé :
(e) en mettant en contact ledit surnageant avec un premier et un second anticorps détectables, ledit premier anticorps détectable étant immunoréactif uniquement avec l'isoforme de 80 kDa de la protéinase µ-PNAC et le second anticorps détectable étant immunoréactif avec les isoformes de 76 kDa, de 78 kDa et de 80 kDa de la protéinase µ-PNAC;
(f) en détectant la quantité de premier et second anticorps détectables lié aux isoformes respectives de la protéinase µ-PNAC pour donner un signal relatif;
(g) en divisant le signal relatif correspondant aux isoformes de 76 kDa, 78 kDa et 80 kDa par le signal relatif correspondant de l'isoforme de 80 kDa pour donner ledit rapport.

42. Méthode selon la revendication 41, dans laquelle ledit anticorps détectable est marqué de manière détectable et la quantité d'anticorps détectable est déterminée dans l'étape (f) en détectant le marqueur.

43. Méthode selon la revendication 41, dans laquelle lesdits premier et second anticorps détectables sont marqués de manière détectable avec deux marqueurs différents.
